# EUROPEAN PATENT APPLICATION

(11) **EP 4 324 461 A1**
(43) Date of publication of application: **21.02.2024**
(21) Application number: 23461638.1
(22) Date of filing: 19.08.2023
(51) Int. Cl.: A61K 31/165, A61K 31/435, A61P 17/00

(54) **(E)-N-CINAMOYLAMINOALKANOLES WITH MELANOGENESIS INHIBITORY ACTIVITY**

(30) Priority: 19.08.2022 PL 44204622
(71) Applicant: UNIWERSYTET JAGIELLONSKI, 31-007 Kraków (PL)
(72) Inventor: Gunia-Krzyzak, Agnieszka, 32-020 Sulków (PL); Popiól, Justyna, 31-831 Kraków (PL); Sloczynska, Karolina, 32-007 Zabierzów Bochenski (PL); Zelaszczyk, Dorota, 31-615 Kraków (PL); Wójcik-Pszczola, Katarzyna, 32-067 Tenczynek (PL); Koczurkiewicz-Adamczyk, Paulina, 32-070 Dabrowa Szlachecka (PL); Pekala, Elzbieta, 31-416 Kraków (PL); Marona, Henryk, 31-867 Kraków (PL); Borczuch-Kostanska, Magdalena, 31-128 Kraków (PL)
(74) Representative: Witek, Rafal

(57) **Abstract**

Uses of (*E*)-*N*-cinnamoylaminoalkanols for the preparation of medicinal or cosmetic products are disclosed.

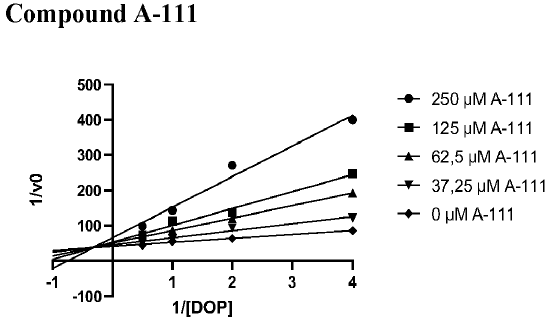

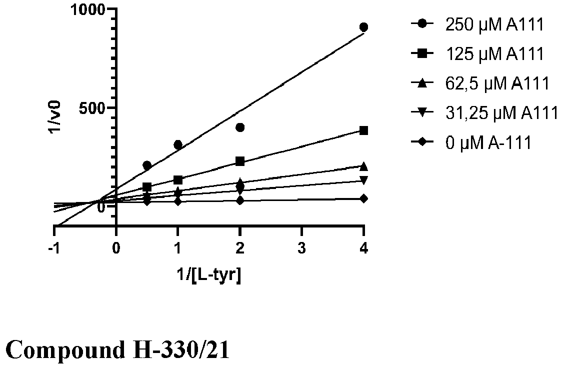

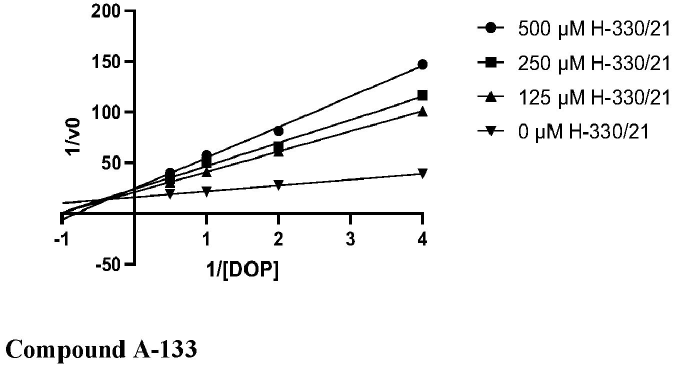

## Description

The object of the invention is the use of (*E*)-*N*-cinnamoylaminoalkanols for the production of medicinal or cosmetic products. The compounds according to the invention have the activity of inhibiting the melanogenesis process, which allows them to be used especially in products used to support the therapy of pigmentation disorders, as well as to improve and unify the skin tone.

### State of the art

Discoloration, also known as pigmentation disorders, arise as a result of disturbances in the synthesis and/or deposition of the pigment (melanin) in the skin. Melanin in the skin is synthesized in the so-called melanogenesis process, which is multi-step and includes both enzymatically catalyzed steps and chemical reactions. The enzyme of the greatest importance for melanogenesis is tyrosinase, which catalyzes its first steps, i.e. hydroxylation and/or oxidation of L-tyrosine and L-dihydroxyphenylalanine (L-DOPA) to dopaquinone.

Hyperpigmentation is a special type of discoloration resulting from excessive accumulation of melanin in specific places in the skin. It includes e.g. melasma, lentigines and postinflammatory, hormonal and solar hyperpigmentations [1,2]. The available studies indicate that the incidence of pigmentation disorders may be as high as 80% in a given population, with dark-skinned people (Fitzpatrick skin phototypes IV-VI) being a particularly high-risk group. This problem may still be underestimated due to the variety of pigmentation changes, as well as the fact that people affected by these diseases do not always consult a doctor or a cosmetologist.

Currently available methods of supporting the treatment of hyperpigmentation include, among others, light therapy with a specific wavelength and laser therapy, chemical peels, as well as the external use of melanogenesis inhibitors, the so-called skin whitening agents [4,5].

Certain cinnamic acid derivatives having tyrosinase and/or melanogenesis inhibitory activity are known in the art (databases: Chemical Abstracts - SciFinder, Google Patents, PubChem).

**Cinnamic acid (Scheme 1a)** has the activity of a non-competitive inhibitor of tyrosinase diphenolase activity (IC₅₀=2.1 mM). 4-Hydroxycinnamic acid (**Scheme 1b**) and 4-methoxycinnamic acid (**Scheme 1c**) exhibited even better activity in this respect, i.e. IC₅₀=0.5 and IC₅₀=0.42 mM, respectively [6]. 2-Chlorocinnamic acid (IC₅₀=0.765 mM) and 2,4-dichlorocinnamic acid (IC₅₀=0.295 mM) exhibited a reversible non-competitive mechanism of tyrosinase inhibition [7]. 4-Hydroxycinnamic acid has been tested in human clinical trials in formulations that inhibit erythema and excessive pigmentation after exposure to ultraviolet radiation. A 7-day application of a cream containing 4-hydroxycinnamic acid to the skin before exposure to UV radiation inhibited erythema formation by 77% compared to untreated skin. In the case of application of the cream after exposure to UV radiation, the effect on pigmentation was less noticeable. However, the measurement of the amount of melanin showed its reduction up to 70 days in the places where the cream was applied compared to the places where no preparation was applied [8].

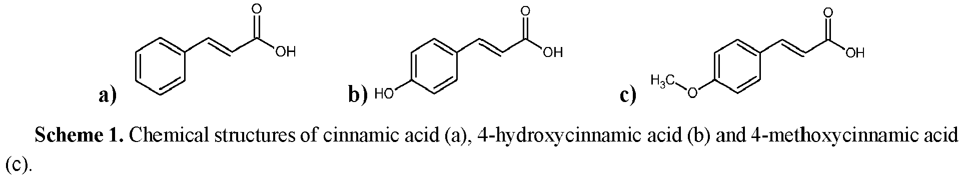

Also some synthetic cinnamic acid derivatives have been described as tyrosinase and/or melanogenesis inhibitors.

Compounds with the general formula shown in **Scheme 2,** which are derivatives of **α-phenylcinnamic acid,** are the object of a **patent** concerning their use as skin brightening, whitening and/or depigmenting compounds [9].

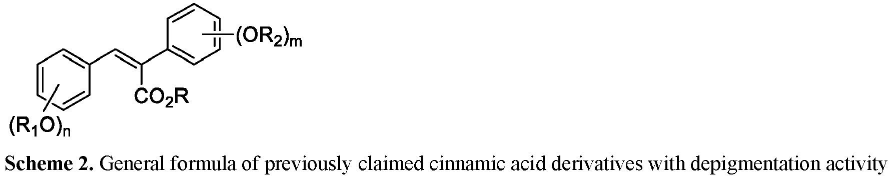

Ullach *et al.* described a series of amide derivatives of cinnamic acid with tyrosinase and/or melanin synthesis inhibitory activity in the B16-F10 murine melanoma cell line. The tested compounds contained a hydroxyl group in the phenyl ring, so their ability to inhibit tyrosinase *in vitro* could be due to their antioxidant activity. The most active compounds were **4, 9, 14** and **19** (**Scheme 3**) 10.

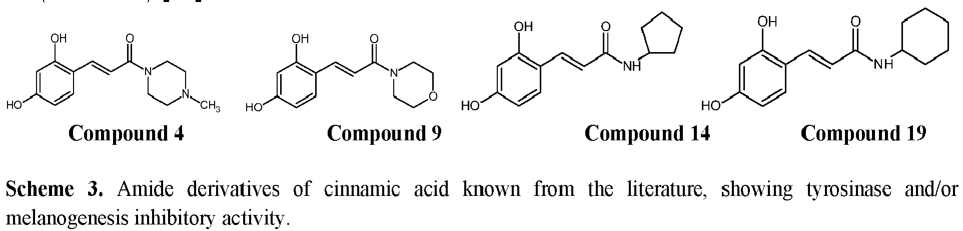

Fan *et al.* described a series of amide derivatives of cinnamic acid with an amino acid ester component. These compounds exhibited antioxidant and tyrosinase inhibitory activity. For example, *N*-[3-(4-hydroxy-3-methoxyphenyl)-1-oxo-2-propen-1-yl]-L-phenylalanine ethyl ester, i.e. the compound shown in **Scheme 4** inhibited tyrosinase with IC₅₀=0.185±0.005 µM, showing a mixed type of inhibition [11].

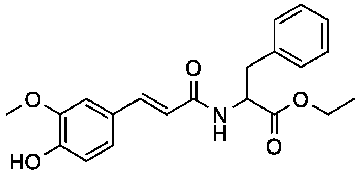

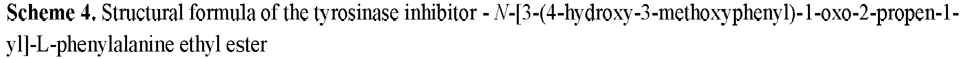

### The essence of the invention

Despite the solutions described above, there is still a need for inhibitors of the melanogenesis process that are highly active and at the same time have a favorable safety profile and are suitable for epidermal administration.

The above technical problem is solved by the present invention.

The object of the invention is compounds and applications defined in the appended claims.

It was unexpectedly found, based on very favorable results of *in vitro* biological tests, that the (*E*)-*N*-cinnamoylaminoalkanols (amide derivatives of cinnamic acid), which are the object to the invention, exhibit: high activity in inhibiting the melanogenesis process, a favorable safety profile and optimal bioavailability after external application.

The compounds according to the invention, unlike many known melanogenesis inhibitors from the group of cinnamic acid derivatives, do not have phenolic groups in their structure. Therefore, their mechanism of inhibiting melanin synthesis is probably not due to antioxidant activity, and thus to the reduction of dopaquinone produced in the enzymatic reaction, which is characteristic of compounds having one or several phenolic groups.

The presented amide derivatives of cinnamic acid are suitable for the production of cosmetic and/or medicinal products, especially those used to support the treatment of discolorations, as well as to improve and unify skin tone. According to the nomenclature of cosmetic raw materials, the substances referred to above could be used in particular in cosmetics as "depigmenting" or "bleaching" raw materials.

### Detailed description of the invention

Therefore, the object of the invention are the applications of (E)-N-cinnamoylaminoalkanols (amide derivatives of cinnamic acid of the general formula 1, in which R1 is an aminoalkanol, and R2 is a hydrogen atom, a chlorine atom, two chlorine atoms, a methyl group in the appropriate position of the phenyl ring, preferably in position 4 or 2,4, preferably with E configuration of a double bond) with melanogenesis inhibitory activity for the production of cosmetic or medicinal products (**Formula 1, Table 1**).

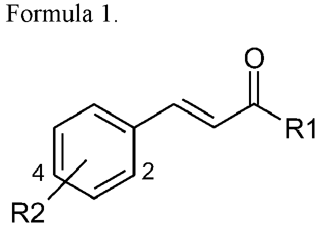

| **R1** | **R2** |
|---|---|
| 6-aminohexan-1-ol | -H |
| 5-aminopentan-1-ol | -Cl |
| 4-aminobutan-1-ol | -diCl |
| 3-aminopropan-1-ol | -CH₃ |
| 2-aminoetan-1-ol | |
| (*R*,*S*)-2-aminopropan-1-ol | |
| (*R*,*5*)-2-aminobutan-1-ol | |
| (*R*,*S*)-1-aminobutan-2-ol | |
| (*R*,*S*)-2-amino-3-methylobutan-1-ol | |
| 4-hydroxypiperidine | |

The invention is illustrated by the following examples.

**Table 1. Symbols, structures and names of compounds.**

| No. | Symbol | Structure and name |
|---|---|---|
| 1 | **H-425/21** | 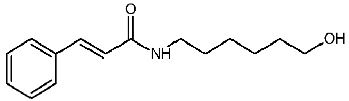 CAS: 605663-05-6 (applies to a compound with an unspecified double bond configuration) |
| | | (*E*)-*N*-(6-hydroxyhexyl)cinnamic acid amide |
| 2 | **H-426/21** | 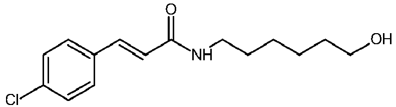 CAS: 1922243-17-1 (applies to a compound with an unspecified double bond configuration) |
| | | (2*E*)-3-(4-chlorophenyl)-*N*-(6-hydroxyhexyl)prop-2-enamide |
| | | (*E*)-3-(4-chlorophenyl)-*N*-(6-hydroxyhexyl)acrylamide |
| 3 | **A-133** | 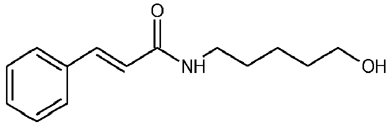 CAS: 113297-90-8 (applies to a compound with *E* configuration) |
| | | (2*E*)-N-(5-hydroxypentyl)-3-phenylprop-2-enamide |
| | | *N*-(5-hydroxypentyl)cinnamic acid amide |
| 4 | **A-111** | 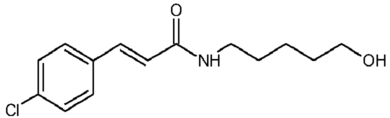 |
| | | CAS: 2075715-66-9 (applies to a compound with *E* configuration) |
| | | (2*E*)-3-(4-chlorophenyl)-*N*-(5-hydroxypentyl)prop-2-enamide |
| | | (*E*)-3-(4- chlorophenyl)-*N*-(5-hydroxypentyl)acrylamide |
| 5 | **H-297/20** | 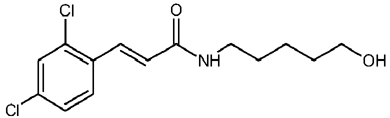 |
| | | CAS: 1966161-71-6 (applies to a compound with an unspecified double bond configuration) |
| | | (2*E*)-3-(2,4-dichlorophenyl)-*N*-(5-hydroxypentyl)prop-2-enamide |
| | | (*E*)-3-(2,4-dichlorophenyl)-*N*-(5-hydroxypentyl)acrylamide |
| 6 | **H-415/21** | 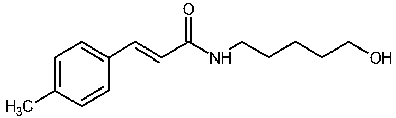 |
| | | CAS: 1799238-85-9 (applies to a compound with an unspecified double bond configuration) |
| | | (2*E*)-*N*-(5-hydroxypentyl)-3-(4-methylphenyl)prop-2-enamide |
| | | (*E*)-*N*-(5-hydroxypentyl)-3-(*p*-tolyl)acrylamide |
| 7 | **A-43** | 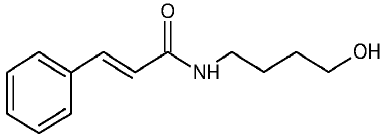 CAS: 2636031-47-3 (applies to a compound with E configuration) |
| | | CAS: 956585-61-8 (applies to a compound with an unspecified double bond configuration) |
| | | (2*E*)-*N*-(4-hydroxybutyl)-3-phenylprop-2-enamide |
| | | *N*-(4-hydroxybutyl)cinnamic acid amide |
| 8 | **H-331/21** | 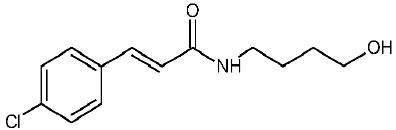 |
| | | CAS: 1982458-62-7 (applies to a compound with an unspecified double bond configuration) |
| | | (2*E*)-3-(4-chlorophenyl)-*N*-(4-hydroxybutyl)prop-2-enamide |
| | | (*E*)-3-(4-chlorophenyl)-*N*-(4-hydroxybutyl)acrylamide |
| 9 | **H-298/20** | 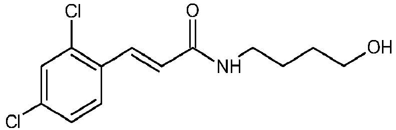 |
| | | CAS: 1148155-59-2 (applies to a compound with an unspecified double bond configuration) |
| | | (2*E*)-3-(2,4-dichlorophenyl)-*N*-(4-hydroxybutyl)prop-2-enamide |
| | | (*E*)-3-(2,4-dichlorophenyl)-*N*-(4-hydroxybutyl)acrylamide |
| 10 | **H-411/21** | 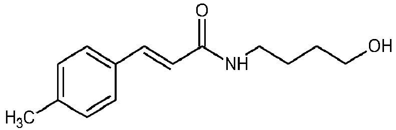 |
| | | CAS: 1982415-19-9 (applies to a compound with an unspecified double bond configuration) |
| | | (2*E*)-*N*-(4-hydroxybutyl)-3-(4-methylphenyl)prop-2-enamide |
| | | (*E*)-*N*-(4-hydroxybutyl)-3-(*p*-tolyl)acrylamide |
| 11 | **KM-634** | 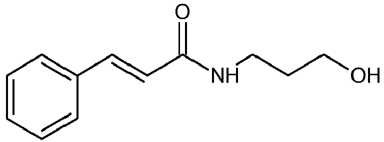 |
| | | CAS: 1206784-23-7 (applies to a compound with E configuration) |
| | | CAS: 30687-13-9 (applies to a compound with an unspecified double bond configuration) |
| | | (2*E*)-*N*-(3-hydroxypropyl)-3-phenylprop-2-enamide |
| | | *N*-(3-hydroxypropyl)cinnamic acid amide |
| 12 | **H-330/21** | 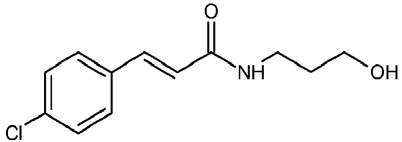 |
| | | CAS: 193219-51-1 (applies to a compound with E configuration) |
| | | CAS: 43196-33-4 (applies to a compound with an unspecified double bond configuration) |
| | | (2*E*)-3-(4-chlorophenyl)-*N*-(3-hydroxypropyl)prop-2-enamide |
| | | (*E*)-3-(4-chlorophenyl)-*N*-(3-hydroxypropyl)acrylamide |
| 13 | **H-296/20** | 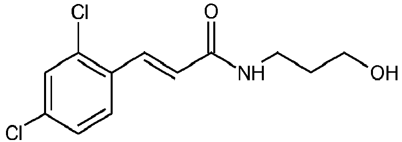 |
| | | CAS: 1158109-67-1 (applies to a compound with an unspecified double bond configuration) |
| | | (2*E*)-3-(2,4-dichlorophenyl)-*N*-(3-hydroxypropyl)prop-2-enamide |
| | | (*E*)-3-(2,4-dichlorophenyl)-*N*-(3-hydroxypropyl)acrylamide |
| 14 | **H-410/21** | 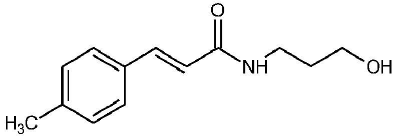 |
| | | CAS: 1158121-14-2 (applies to a compound with an unspecified double bond configuration) |
| | | (2*E*)-*N*-(3-hydroxypropyl)-3-(4-methylphenyl)prop-2-enamide |
| | | (*E*)-*N*-(3-hydroxypropyl)-3-(*p*-tolyl)acrylamide |
| 15 | **H-429/21** | 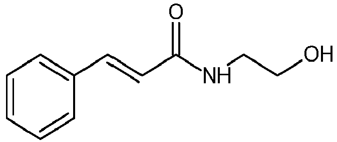 |
| | | CAS: 35241-61-3 (applies to a compound with E configuration) |
| | | CAS: 6961-46-2 (applies to a compound with an unspecified double bond configuration) |
| | | (2*E*)-*N*-(2-hydroxyethyl)-3-phenylprop-2-enamide |
| | | *N*-(2-hydroxyethyl)cinnamic acid amide |
| | | Synonym: idrocilamide |
| 16 | **H-427/21** | 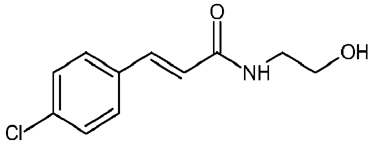 |
| | | CAS: 1187423-32-0 (applies to a compound with E configuration) |
| | | CAS: 30687-04-8 (applies to a compound with an unspecified double bond configuration) |
| | | (2*E*)-3-(4-chlorophenyl)-*N*-(2-hydroxyethyl)prop-2-enamide |
| | | (*E*)-3-(4-chlorophenyl)-*N*-(2-hydroxyethyl)acrylamide |
| 17 | **A-67** | 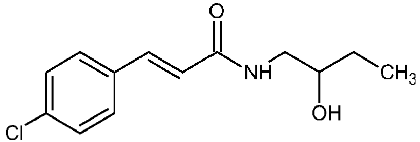 |
| | | CAS: 2075715-55-6 (applies to a compound with *E* configuration) |
| | | CAS: 1786034-81-8 (applies to a compound with an unspecified double bond configuration) |
| | | (2*E*)-3-(4-chlorophenyl)-*N*-(2-hydroxybutyl)prop-2-enamide |
| | | (*E*)-3-(4-chlorophenyl)-*N*-(2-hydroxybutyl)acrylamide |
| 18 | **H-320/21** | 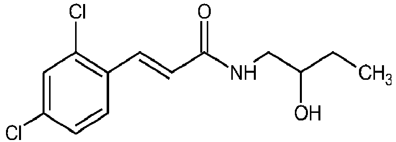 |
| | | CAS: 1785808-25-4 (applies to a compound with an unspecified double bond configuration) |
| | | (2*E*)-3-(2,4-dichlorophenyl)-*N*-(2-hydroxybutyl)prop-2-enamide |
| | | (*E*)-3-(2,4-dichlorophenyl)-*N*-(2-hydroxybutyl)acrylamide |
| 19 | **A-63** | 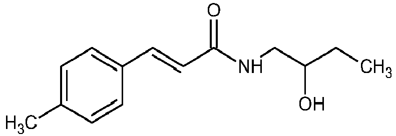 |
| | | CAS: 2075715-58-9 (applies to a compound with *E, R,S* configuration) |
| | | CAS: 1786034-81-8 (applies to a compound with an unspecified double bond configuration) |
| | | (2*E*)-*N*-(2-hydroxybutyl)-3-(4-methylphenyl)prop-2-enamide |
| | | (*E*)-*N*-(2-hydroxybutyl)-3-(*p*-tolyl)acrylamide |
| 20 | **A-102** | 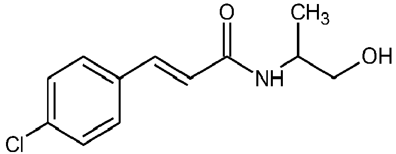 |
| | | CAS: 2075715-22-7 (applies to a compound with *E, R,S* configuration) |
| | | CAS: 1158109-54-6 (applies to a compound with an unspecified double bond configuration) |
| | | (2*E*)-3-(4-chlorophenyl)-*N*-(1-hydroxypropan-2-yl)prop-2-enamide |
| | | (*E*)-3-(4-chlorophenyl)-*N*-(1-hydroxypropan-2-yl)acrylamide |
| 21 | **A-69** | 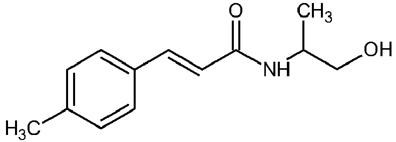 |
| | | CAS: 2075715-25-0 (applies to a compound with *E, R,S* configuration) |
| | | CAS: 1158094-03-1 (applies to a compound with an unspecified double bond configuration) |
| | | (2*E*)-*N*-(1-hydroxypropan-2-yl)-3-(4-methylphenyl)prop-2-enamide |
| | | (*E*)-*N*-(1-hydroxypropan-2-yl)-3-(*p*-tolyl)acrylamide |
| 22 | **A-01** | 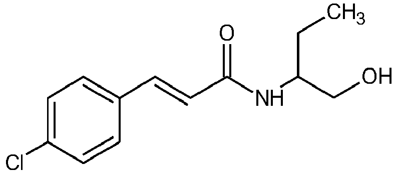 |
| | | CAS: 1609486-37-4 (applies to a compound with *E, R,S* configuration) |
| | | CAS: 906728-38-9 (applies to a compound with an unspecified double bond configuration) |
| | | (2*E*)-3-(4-chlorophenyl)-*N*-(1-hydroxybutan-2-yl)prop-2-enamide |
| | | (*E*)-3-(4-chlorophenyl)-*N*-(1-hydroxybutan-2-yl)acrylamide |
| 23 | **H-285/20** | 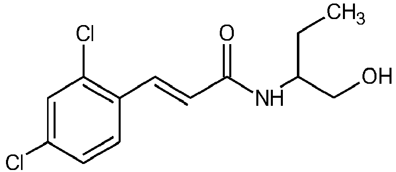 |
| | | CAS: 1158140-78-3 (applies to a compound with an unspecified double bond configuration) |
| | | (2*E*)-3-(2,4-dichlorophenyl)-*N*-(1-hydroxybutan-2-yl)prop-2-enamide |
| | | (*E*)-3-(2,4-dichlorophenyl)-*N*-(1-hydroxybutan-2-yl)acrylamide |
| 24 | **H-409/21** | 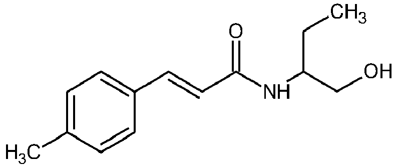 |
| | | CAS: 2075715-43-2 (applies to a compound with *E, R,S* configuration) |
| | | CAS: 1158104-34-7 (applies to a compound with an unspecified double bond configuration) |
| | | (2*E*)-*N*-(1-hydroxybutan-2-yl)-3-(4-methylphenyl)prop-2-enamide |
| | | (*E*)-*N*-(1-hydroxybutan-2-yl)-3-(*p*-tolyl)acrylamide |
| 25 | **A-106** | 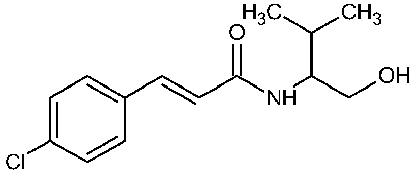 |
| | | CAS: 1562269-25-3 (applies to a compound with an unspecified double bond configuration) |
| | | (2*E*)-3-(4-chlorophenyl)-*N*-(1-hydroxy-3-methylbutan-2-yl)prop-2-enamide |
| | | (*E*)-3-(4-chlorophenyl)-*N*-(1-hydroxy-3-methylbutan-2-yl)acrylamide |
| 26 | **A-99** | 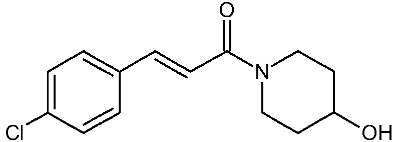 |
| | | CAS: 31362-74-0 (applies to a compound with an unspecified double bond configuration) |
| | | (2*E*)-3-(4-chlorophenyl)-1-(4-hydroxypiperidin-1-yl)prop-2-en-1-one |
| | | (*E*)-3-(4-chlorophenyl)-1-(4-hydroxypiperidin-1-yl)prop-2-en-1-one |
| 27 | **H-310/20** | 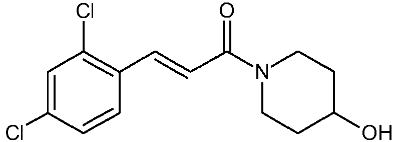 |
| | | CAS: 1158135-40-0 (applies to a compound with an unspecified double bond configuration) |
| | | (2*E*)-3-(2,4-dichlorophenyl)-1-(4-hydroxypiperidin-1-yl)prop-2-en-1-one |
| | | (*E*)-3-(2,4-dichlorophenyl)-1-(4-hydroxypiperidin-1-yl)prop-2-en-1-one |

### Example 1. Compound H-425/21

50 ml of distilled water was added to a 250 mL conical flask. 2.07 g (0.015 mol) of K₂CO₃ was dissolved in it, and then 0.01 mol, i.e. 1.17 g of 6-aminohexan-1-ol, was added, followed by 10 mL of toluene. The flask was placed on a magnetic stirrer at room temperature and a solution of 1.67 g (0.01 mol) of (E)-cinnamic acid chloride in 30 mL of toluene was added dropwise. The contents of the flask were constantly stirred, the addition time was 20 minutes. After the process was completed, the contents of the flask were cooled for 4 hours, the resulting precipitate was filtered off, and then rinsed with 50 mL of 5% HCl, 50 mL of 5% NaOH and 100 mL of distilled water. The precipitate was dried and crystallized from n-hexane:acetone (5:1 v/v). A compound of formula 1 was obtained, in which R2=H, R1=6-aminohexan-1-ol.

### Example 2. Compound H-426/21

### Step I. Preparation of the acid chloride.

5 g of (E)-4-chlorocinnamic acid was added to a 250 ml round-bottom flask, then 30 mL of toluene and 5 mL of thionyl chloride (d=1.63 g/mL) were added. It was heated under reflux for 4 hours, then concentrated to an oily residue, to which 10 mL of toluene was added twice, distilling it off each time. The residue was crystallized from n-hexane. The resulting product was used in the next reaction.

### Step II. N-acylation in a two-phase medium.

50 ml of 5% K₂CO₃ was prepared in a 250 mL conical flask. 1.17 g (0.01 mol) of 6-aminohexan-1-ol and 10 mL of toluene were added. The flask was placed on a magnetic stirrer at room temperature, and a solution of 2.01 g (0.01 mol) of (*E*)-4-chlorocinnamic acid chloride in 40 mL of toluene was then added dropwise to the flask. The addition time was about 20 minutes, the contents of the flask were constantly stirred. After the process was completed, the contents of the flask were cooled for 4 hours, the resulting precipitate was filtered off, and then rinsed with 50 mL of 5% HCl, 50 mL of 5% NaOH and 100 mL of distilled water. The precipitate was dried and crystallized from n-hexane:acetone (5:1 v/v). A compound of formula 1 was obtained, in which R2 = 4-Cl, R1 = 6-aminohexan-1-ol.

The other compounds for which R2 = 4-Cl were obtained analogously using appropriate reactants:
Step 1. (*E*)-4-chlorocinnamic acid
Step 2.
   1. (*E*)-4-chlorocinnamic acid chloride
   2. 5-aminopentan-1-ol for compound **H-331/21**
      *R*,*S*-3-methyl-2-aminobutan-1-ol for compound **A-106**
      4-hydroxypiperidine for compound **A-99**

### Example 3. Compound H-297/20

### Step I. Preparation of the acid chloride.

5 g of (*E*)-2,4-dichlorocinnamic acid was added to a 250 ml round-bottom flask, then 30 mL of toluene and 5 mL of thionyl chloride (d=1.63 g/mL) were added. It was heated under reflux for 4 hours, then concentrated to an oily residue, to which 10 mL of toluene was added twice, distilling it off each time. The residue was crystallized from n-hexane. The resulting product was used in the next reaction.

### Step II. N-acylation in a two-phase medium.

50 ml of 5% K₂CO₃ was prepared in a 250 mL conical flask. 1.03 g (0.01 mol) of 5-aminopenta-1-ol and 10 mL of toluene were added. The flask was placed on a magnetic stirrer at room temperature, and a solution of 2.36 g (0.01 mol) of (*E*)-2,4-dichlorocinnamic acid chloride in 40 mL of toluene was then added dropwise to the flask. The addition time was about 20 minutes, the contents of the flask were constantly stirred. After the process was completed, the contents of the flask were cooled for 4 hours, the resulting precipitate was filtered off, and then rinsed with 50 mL of 5% HCl, 50 mL of 5% NaOH and 100 mL of distilled water. The precipitate was dried and crystallized from n-hexane:acetone (5:1 v/v). A compound of formula 1 was obtained, in which R2 = 2,4-diCl, R1 = 5-aminopentan-1-ol.

The other compounds for which R2 = 2,4-diCl were obtained analogously using appropriate reactants:
Step 1. (*E*)-2,4-dichlorocinnamic acid
Step 2.
   1. (*E*)-2,4-dichlorocinnamic acid chloride
   2. 4-aminobutan-1-ol for compound **H-298/20**
   3-aminopropan-1-ol for compound **H-296/20**
      *R*,*S*-1-aminobutan-2-ol oxalate for compound **H-320/21**
      *R,S*-2-aminobutan-1-ol for the compound **H-285/20**
      4-hydroxypiperidine for compound **H-310/20**

### Example 4. Compound H-415/21

### Step I. Preparation of the acid chloride.

5 g of (*E*)-4-methylcinnamic acid was added to a 250 ml round-bottom flask, then 30 mL of toluene and 5 mL of thionyl chloride were added (d=1.63 g/mL). It was heated under reflux for 4 hours, then concentrated to an oily residue, to which 10 mL of toluene was added twice, distilling it off each time. The residue was crystallized from n-hexane. The resulting product was used in the next reaction.

### Step II. N-acylation in a two-phase medium.

50 ml of 5% K₂CO₃ was prepared in a 250 mL conical flask. 1.03 g (0.01 mol) of 5-aminopenta-1-ol and 10 mL of toluene were added. The flask was placed on a magnetic stirrer at room temperature, and a solution of 1.81 g (0.01 mol) of (*E*)-4-methylcinnamic acid chloride in 40 mL of toluene was added dropwise to the flask. The addition time was about 20 minutes, the contents of the flask were constantly stirred. After the process was completed, the contents of the flask were cooled for 4 hours, the resulting precipitate was filtered off, and then rinsed with 50 mL of 5% HCl, 50 mL of 5% NaOH and 100 mL of distilled water. The precipitate was dried and crystallized from n-hexane:acetone (5:1 v/v). A compound of formula 1 was obtained, in which R2 = 4-CH₃, R1 = 5-aminopentan-1-ol.

The other compounds for which R2 = 4-CH₃ were obtained analogously using appropriate reactants:
Step 1. (*E*)-4-methylcinnamic acid
Step 2.
   1. (*E*)-4-methylcinnamic acid chloride
   2. 4-aminobutan-1-ol for compound **H-411/21**
   3-aminopropan-1-ol for compound **H-410/21**

Confirmation of the identity and purity of the obtained compounds consisted in the registration and interpretation of ¹H NMR and ¹³C NMR spectra (the spectra were recorded using an ECZR 500 MHz (JEOL) series FT-NMR spectrophotometer), as well as the registration and interpretation of LC-MS spectra (the spectra were recorded using Acquita UPLC system (Waters) coupled with a Waters TQD mass spectrometer). The melting point was measured using the open capillary method in the IA9300 device (Electrothermal). The lipophilicity coefficient R_{M0} was determined by reversed phase thin layer chromatography. In the study, aluminum plates 20x20 cm covered with modified silica gel RP-18 F254 (Merck) were used as the solid phase. The mobile phase consisted of solutions of methanol and 100 mM phosphate buffer (pH = 7.4), characterized by a variable methanol content ranging from 95 to 65% (v/v). The tested compounds were dissolved in methanol to obtain solutions with a concentration of 1 mg/mL. Then, 5 µL of the solutions prepared in this way were transferred to the starting line on aluminum plates, which were then placed in chromatographic chambers previously saturated with mobile phase vapors for at least 1 hour. All activities, including the development of chromatograms, were carried out at room temperature. After developing the chromatograms to approximately 1-2 cm from the end of the plate, the plates were dried and then the distance traveled by the tested compounds was visualized under UV light (λ = 254 nm). The experiment was performed in duplicate for the specified conditions. For all samples, R_{f} was calculated according to the formula R_{f} = a/b, where a - the distance traveled by the tested compound, b - the distance traveled by the mobile phase. Then, the R_{M} parameter was calculated for all samples according to the formula R_{M} = log(1/R_{f} - 1), obtaining a linear relationship between the R_{M} values and the methanol concentration in the mobile phase. The logP coefficient was also calculated using the following computer programs: ACD/ChemSketch 2020.1.2 and ChemDraw 20.0.0.41.

**Table 2. The physicochemical data of the compounds are presented below**

| No. | Symbol | Physicochemical data |
|---|---|---|
| 1 | **H-425/21** | M=247.33, [M+H]⁺ calculated 248.16, [M+H]⁺ found 248.32 (100%); Mp=93-95 °C |
| | | ¹H NMR (500 MHz, DMSO-*d*₆) δ ppm 8.05 (t, *J*=5.7 Hz, 1H, NH), 7.53 - 7.49 (m, 2H, Ar-H), 7.39 - 7.30 (m, 4H, Ar-H, Ar-CH=), 6.60 (d, *J*=15.7 Hz, 1H, Ar-CH=CH), 4.31 (t, *J*=5.2 Hz, 1H, OH), 3.35 (q, *J*=6.5, *J*=5.3 Hz, 2H, CH₂-OH), 3.13 (q, *J*=6.9, *J*=6.0, 2H, CH₂-OH), 1.45 - 1.34 (m, 4H, CH₂-CH₂-OH, NH-CH₂-CH₂), 1.29 - 1.23 (m, 2H, CH₂-CH₂-CH₂-CH₂) |
| | | ¹³C NMR (126 MHz, DMSO-*d*₆) δ ppm 165.29 (C=O), 138.87 (Ar-CH=), 135.51 (Ar(1)), 129.87 (Ar(4)), 129.44 (2C, Ar(3,5)), 127.98 (2C, Ar(2,6)), 122.91 (Ar-CH=CH), 61.19 (CH₂-OH), 39.21 (NH-CH₂), 33.03 (CH₂-CH₂-OH), 29.75 (NH-CH₂-CH₂), 26.94 (CH₂-CH₂-CH₂), 25.81 (CH₂-CH₂-CH₂) |
| | | R_{M0}=2.40; logP (ChemSketch)=2.10+/- 0.33; logP (ChemDraw)=2.39; ClogP (ChemDraw)=2.46 |
| 2 | **H-426/21** | M=281.78 [M+H]⁺ calculated 282.13, [M+H]⁺ found 282.35 (100%), Mp=129-131 °C |
| | | ¹H NMR (500 MHz, DMSO-*d*₆) δ ppm 8.06 (t, *J*=5.7 Hz, 1H, NH), 7.56-7.51 (m, 2H, Ar-H), 7.45-7.40 (m, 2H, Ar-H), 7.36 (d, *J*=15.7 Hz, Ar-CH=), 6.59 (d, *J*=15.8 Hz, 1H, Ar-CH=CH), 4.31 (t, *J*=5.2 Hz, 1H, OH), 3.34 (q, *J*=6.5, *J*=6.3 Hz, 2H, CH₂-OH), 3.13 (q, *J*=6.8, *J*=6.1, 2H, CH₂-OH), 1.44 - 1.33 (m, 4H, CH₂-CHz-OH, NH-CH₂-CH₂), 1.29 - 1.22 (m, 2H, CH₂-CH₂-CH₂-CH₂) |
| | | ¹³C NMR (126 MHz, DMSO-*d*₆) δ ppm 165.08 (C=O), 137.53 (Ar-CH=), 134.48 (Ar(C4)), 134.28 (Ar(C1)), 129.69 (2C, Ar(C3,C5), 129.48 (2C, Ar(C2,C6), 123.72 (Ar-CH=CH), 61.18 (CH₂-OH), 39.22 (NH-CH₂), 33.03 (CH₂-CH₂-OH), 29.73 (NH-CH₂-CH₂), 26.93 (CH₂-CH₂-CH₂), 25.80 (CH₂-CH₂-CH₂) |
| | | R_{M0}=3.02; logP (ChemSketch)=2.63+/- 0.35; logP (ChemDraw)=2.95; ClogP (ChemDraw)=3.18 |
| 3 | **A-133** | R_{M0}=2.15; logP (ChemSketch)=1.57+/- 0.33; logP (ChemDraw)=1.98; ClogP (ChemDraw)=1.93 |
| 4 | **A-111** | R_{M0}=2.79; logP (ChemSketch)=2.09+/- 0.35; logP (ChemDraw)=2.54; ClogP (ChemDraw)=2.65 |
| 5 | **H-297/20** | M=302.20 [M+H]⁺ calculated 302.07, [M+H]⁺ found 302.25 (100%), Mp=132-134 °C |
| | | ¹H NMR (500 MHz, DMSO-*d*₆) δ ppm 8.18 (t, *J*=5.7 Hz, 1H, NH), 7.68 - 7.63 (m, 2H, Ar-H), 7.60 (d, *J*=15.8 Hz, 1H, Ar-CH=), 7.45 (dd, *J*=6.9, *J*=2.3 Hz, 1H, Ar-H), 6.64 (d, *J*=15.8 Hz, 1H, Ar-CH=CH), 4.34 (t, *J*=5.2 Hz, 1H, OH), 3.37 - 3.31 (m, 2H, CH₂-OH), 3.16 - 3.09 (m, 1H, NH-CH₂), 1.45 - 1.35 (m, 4H, CH₂-CH₂-OH, NH-CH₂-CH₂), 1.31 - 1.23 (m, 2H, CH₂-CH₂-CH₂) |
| | | ¹³C NMR (126 MHz, DMSO-*d*₆) δ ppm 164.60 (C=O), 134.87 (Ar-CH=), 134.54 (Ar(4)), 133.09 (Ar(2)), 132.41 (Ar(3)), 129.92 (Ar(1)), 129.29 (Ar(6)), 128.55 (Ar(5)), 126.64 (Ar-CH=CH), 61.14 (CH₂-OH), 39.36 (NH-CH₂), 32.74 (CH₂-CH₂-OH), 29.50 (NH-CH₂-CH₂), 23.57 (CH₂-CH₂-CH₂) |
| | | R_{M0}=3.08; logP (ChemSketch)= 2.49+/- 0.37; logP (ChemDraw)= 3.09; ClogP (ChemDraw)=3.36 |
| 6 | **H-415/21** | M=247.33 [M+H]⁺ calculated 248.16 [M+H]⁺ found 248.32 (100%), Mp=101-103 °C |
| | | ¹H NMR (500 MHz, DMSO-*d*₆) δ ppm 8.00 (t, *J*=5.7 Hz, 1H, NH), 7.41-7.38 (m, 2H, Ar-H), 7.33 (d, *J*=16.0 Hz, Ar-CH=), 7.19-7.15 (m, 2H, Ar-H), 7.53 (d, *J*=15.8 Hz, 1H, Ar-CH=CH), 4.33 (t, *J*=*5.2* Hz, 1H, OH), 3.35 (td, *J*=6.4, *J=*5*.2* Hz, 2H, CH₂-OH), 3.12 (q, *J*=5.7, *J*=6.9 Hz, 2H, NH-CH₂), 2.27 (s, 3H, CH₃), 1.45 - 1.35 (m, 4H, CH₂-CH₂-OH, NH-CH₂-CH₂), 1.31 - 1.23 (m, 2H, CH₂-CH₂-CH₂) |
| | | ¹³C NMR (126 MHz, DMSO-*d*₆) δ ppm 165.43 (C=O), 139.58 (Ar-CH=), 138.81 (Ar(C4)), 132.75 (Ar(C1)), 130.04 (2C, Ar(C3,C5), 129.95 (2C, Ar(C2,C6), 121.87 (Ar-CH=CH), 61.16 (CH₂-OH), 39.24 (NH-CH₂), 32.78 (CH₂-CH₂-OH), 29.63 (NH-CH₂-CH₂), 23.58 (CH₂-CH₂-CH₂), 21.45 (CH₃) |
| | | R_{M0}=2.49; logP (ChemSketch)=2.03+/- 0.33; logP (ChemDraw)=2.46; ClogP (ChemDraw)=2.43 |
| 7 | **A-43** | R_{M0}=1.60; logP (ChemSketch)=1.34+/- 0.33; logP (ChemDraw)=1.56; ClogP (ChemDraw)=1.41 |
| 8 | **H-331/21** | M=253.72 [M+H]⁺ calculated 254.09, [M+H]⁺ found 254.17 (100%), Mp=151-153 °C |
| | | ¹H NMR (500 MHz, DMSO-*d*₆) δ ppm 8.07 (t, *J*=5,6 Hz, 1H, NH), 7.56-7.51 (m, 2H, Ar-H), 7.45-7.40 (m, 2H, Ar-H), 7.36 (d, *J*=15.8 Hz, Ar-CH=), 6.59 (d, *J*=15.8 Hz, 1H, Ar-CH=CH), 4.37 (t, *J*=5,2 Hz, 1H, OH), 3.37 (q, *J*=6.3, *J*=6.0 Hz, 2H, CH₂-OH), 3.13 (q, *J*=6.6, *J*=6.6 Hz, 2H, CH₂-NH), 1.48 - 1.36 (m, 4H, CH₂-CH₂) |
| | | ¹³C NMR (126 MHz, DMSO-*d*₆) δ ppm 165.09 (C=O), 137.54 (Ar-CH=), 134.48 (Ar(C4)), 134.28 (Ar(C1)), 129.68 (2C, Ar(C3,C5), 129.48 (2C, Ar(C2,C6), 123.73 (Ar-CH=CH), 60.94 (CH₂-OH), 39.14 (CH₂-NH), 30.48 (CH₂-CH₂-OH), 26.36 (NH-CH₂-CH₂) |
| | | R_{M0}=2.14; logP (ChemSketch)=1.87+/- 0.35; logP (ChemDraw)=2.12; ClogP (ChemDraw)=2.12 |
| 9 | **H-298/20** | M=288.17 [M+H]⁺ calculated 288.06, [M+H]⁺ found 288.19 (100%), Mp=138-140 °C |
| | | ¹H NMR (500 MHz, DMSO-*d*₆) δ ppm 8.19 (t, *J*=5.6 Hz, 1H, NH), 7.69 - 7.64 (m, 2H, Ar-H), 7.60 (d, *J*=15.7 Hz, 1H, Ar-CH=), 7.47 - 7.43 (m, 1H, Ar-H), 6.65 (d, *J*=15.5 Hz, 1H, Ar-CH=CH), 4.39 (t, *J*=*5.2* Hz, 1H, OH), 3.37 (q, *J*=6.3 Hz, 2H, CH₂-OH), 3.15 (q, *J*=6.9 Hz, 2H, CH₂-NH), 1.48 - 1.36 (m, 4H, CH₂-CH₂) |
| | | ¹³C NMR (126 MHz, DMSO-*d*₆) δ ppm 164.62 (C=O), 134.88 (Ar-CH=), 134.54 (Ar(4)), 133.11 (Ar(2)), 132.40 (Ar(3)), 129.93 (Ar(1)), 129.29 (Ar(6)), 128.56 (Ar(5)), 126.63 (Ar-CH=CH), 60.92 (CH₂-OH), 39.22 ((CH₂-NH), 30.46 (CH₂-CH₂-OH), 26.29 (NH-CH₂-CH₂) |
| | | R_{M0}=2.78; logP (ChemSketch)=2.26+/- 0.37; logP (ChemDraw)=2.68; ClogP (ChemDraw)=2.83 |
| 10 | **H-411/21** | M=233.31 [M+H]⁺ calculated 234.15 [M+H]⁺ found 234.24 (98%), Mp=125-127 °C |
| | | ¹H NMR (500 MHz, DMSO-*d*₆) δ ppm 8.02 (t, *J*=5.4 Hz, 1H, NH), 7.43 - 7.37 (m, 2H, Ar-H), 7.33 (d, *J*=16.0 Hz, 1H, Ar-CH=), 7.20 - 7.15 (m, 2H, Ar-H), 6.53 (d, *J*=15.8 Hz, 1H, Ar-CH=CH), 4.39 (t, *J*=4.9 Hz, 1H, OH), 3.37 (q, *J*=5.5 Hz, 2H, CH₂-OH), 3.13 (q, *J*=6.5 Hz, 2H, CH₂-NH), 2.27 (s, 3H, CH₃), 1.48 - 1.36 (m, 4H, CH₂-CH₂) |
| | | ¹³C NMR (126 MHz, DMSO-*d*₆) δ ppm 165.45 (C=O), 139.59 (Ar-CH=), 138.82 (Ar(4)), 132.74 (Ar(1)), 130.04 (2C, Ar(C3,C5)), 127.95 (Ar(C2,C6)), 121.87 (Ar-CH=CH), 60.96 (CH₂-OH), 39.10 ((CH₂-NH), 30.50 (CH₂-CH₂-OH), 26.40 (NH-CH₂-CH₂), 21.45 (CH₃) |
| | | R_{M0}=2.27; logP (ChemSketch)=1.80+/- 0.34; logP (ChemDraw)=2.05; ClogP (ChemDraw)=1.90 |
| 11 | **KM-634** | R_{M0}=1.60; logP (ChemSketch)=1.04+/- 0.34; logP (ChemDraw)=1.11; ClogP (ChemDraw)=1.53 |
| 12 | **H-330/21** | R_{M0}=1.94; logP (ChemSketch)=1.56+/- 0.36; logP (ChemDraw)=1.66; ClogP (ChemDraw)=2.24 |
| 13 | **H-296/20** | M=274.14 [M+H]⁺ calculated 274.04, [M+H]⁺ found 274.17 (100%), Mp=114-116 °C |
| | | ¹H NMR (500 MHz, DMSO-*d*₆) δ ppm 8.19 (t, *J*=5.7 Hz, 1H, NH), 7.70 - 7.64 (m, 2H, Ar-H), 7.60 (d, *J*=15.8 Hz, 1H, Ar-CH=), 7.47 - 7.43 (m, 1H, Ar-H), 6.65 (d, *J*=15.8 Hz, 1H, Ar-CH=CH), 4.46 (t, *J*=5.2 Hz, 1H, OH), 3.41 (q, *J*=6.3, *J*=5.4 Hz, 2H, CH₂-OH), 3.20 (q, *J*=6.9, *J*=6.0 Hz, 2H, CH₂-NH), 1.57 (quin, *J*=6.5 Hz, 2H, CH₂-CH₂-CH₂), |
| | | ¹³C NMR (126 MHz, DMSO-*d*₆) δ ppm 164.77 (C=O), 134.89 (Ar-CH=), 134.55 (Ar(4)), 133.13 (Ar(2)), 132.39 (Ar(3)), 129.92 (Ar(1)), 129.30 (Ar(6)), 128.56 (Ar(4)), 126.57 (Ar-CH=CH), 58.92 (CH₂-OH), 36.56 ((CH₂-NH), 32.85 (CH₂-CH₂-CH₂) |
| | | R_{M0}=2.11; logP (ChemSketch)=1.96+/- 0.37; logP (ChemDraw)=2.22; ClogP (ChemDraw)=2.95 |
| 14 | **H-410/21** | M=219.28 [M+H]⁺ calculated 220.13, [M+H]⁺ found 220.28 (99,43%), Mp=101-103 °C |
| | | ¹H NMR (500 MHz, DMSO-*d*₆) δ ppm 8.03 (t, *J*=5.6 Hz, 1H, NH), 7.40 (d, *J*=8.3 Hz, 2H, Ar-H), 7.30 (d, *J*=15.8 Hz, 1H, Ar-CH=), 7.17 (d, *J*=8.0 Hz, 2H, Ar-H), 6.53 (d, .7=15.8 Hz, 1H, Ar-CH=CH), 4.45 (t, *J*=5.5 Hz, 1H, OH), 3.41 (q, *J*=6.0, *J*=5.2 Hz, 2H, CH₂-OH), 3.19 (q, *J*=6.9, *J*=6.0 Hz, 2H, CH₂-NH), 2.27 (s, 3H, CH₃), 1.57 (quin, *J*=6.3 Hz, 2H, CH₂-CH₂-CH₂) |
| | | ¹³C NMR (126 MHz, DMSO-*d*₆) δ ppm 165.64 (C=O), 139.61 (Ar-CH=), 138.88 (Ar(4)), 132.72 (Ar(1)), 130.04 (2C, Ar(3,5)), 127.97 (2C, Ar(2,6)), 121.77 (Ar-CH=CH), 58.94 (CH₂-OH), 36.42 (CH₂-NH), 32.99 (CH₂-CH₃-CH₂), 21.46 (CH₃) |
| | | R_{M0}=2.04; logP (ChemSketch)=1.50+/- 0.34; logP (ChemDraw)=1.59; ClogP (ChemDraw)=2.03 |
| 15 | **H-429/21** | R_{M0}=1.27; logP (ChemSketch)=0.82+/- 0.35; logP (ChemDraw)=1.00; ClogP (ChemDraw)=1.19 |
| 16 | **H-427/21** | R_{M0}=1.93; logP (ChemSketch)=1.35+/- 0.37; logP (ChemDraw)=1.56; ClogP (ChemDraw)=1.91 |
| 17 | **A-67** | R_{M0}=2.46; logP (ChemSketch)= 2.23+/- 0.37; logP (ChemDraw)=2.36; ClogP (ChemDraw)=2.75 |
| 18 | **H-320/21** | M=288.17 [M+H]⁺ calculated 288.06, [M+H]⁺ found 288.13 (100%), Mp=126-128 °C |
| | | ¹H NMR (500 MHz, DMSO-*d*₆) δ ppm 8.16 (t, *J*=5.9 Hz, 1H, NH), 7.68 - 7.63 (m, 2H, Ar-H), 7.60 (d, *J*=15.8 Hz, 1H, Ar-CH=), 7.46 (dd, *J*=8.5, *J*=2.2 Hz, 1H, Ar-H), 6.76 (d, *J*=15.5 Hz, 1H, Ar-CH=CH), 4.71 (t, *J*=5.2 Hz, 1H, OH), 3.44 - 3.37 (m, 1H, CH-OH), 3.24 - 3.17 (m, 1H, CHH-NH), 3.08 - 3.01 (m, 1H, CHH-NH), 1.43 - 1.34 (m, 1H, CHH-CH₃), 1.31 - 1.22 (m, 1H, CHH-CH₃), 0.84 (t, *J*=7.5 Hz, 3H, CH₃) |
| | | ¹³C NMR (126 MHz, DMSO-*d*₆) δ ppm 164.88 (C=O), 134.87 (Ar-CH=), 134.55 (Ar(4)), 133.11 (Ar(2)), 132.42 (Ar(3)), 129.94 (Ar(1)), 129.23 (Ar(6)), 128.58 (Ar(5)), 126.72 (Ar-CH=CH), 70.89 (CH-OH), 45.36 (CH₂-NH), 27.93 (CH₂-CH₃), 10.44 (CH₃) |
| | | R_{M0}=3.08; logP (ChemSketch)= 2.62+/- 0.38; logP (ChemDraw)=2.92; ClogP (ChemDraw)=3.46 |
| 19 | **A-63** | R_{M0}=2.39; logP (ChemSketch)=2.16+/- 0.35; logP (ChemDraw)=2.29; ClogP (ChemDraw)=2.53 |
| 20 | **A-102** | R_{M0}=2.12; logP (ChemSketch)= 1.69+/-0.37; logP (ChemDraw)=1.88; ClogP (ChemDraw)=2.22 |
| 21 | **A-69** | R_{M0}=2.17; logP (ChemSketch)=1.63+/- 0.35; logP (ChemDraw)=1.81; ClogP (ChemDraw)=2.00 |
| 22 | **A-01** | R_{M0}=2.43; logP (ChemSketch)= 2.23+/-0.37; logP (ChemDraw)=2.36; ClogP (ChemDraw)=2.75 |
| 23 | **H-285/20** | M=288.17 [M+H]⁺ calculated 288.06, [M+H]⁺ found 288.02 (100%), Mp=145-147 °C |
| | | ¹H NMR (500 MHz, DMSO-*d*₆) δ ppm 7.93 (d, *J*=8.6 Hz, 1H, NH), 7.67 - 7.64 (m, 2H, Ar-H), 7.60 (d, *J*=15.8 Hz, 1H, Ar-CH=), 7.46 (dd, *J*=6.9, *J*=2.3 Hz, 1H, Ar-H), 6.73 (d, *J*=15.8 Hz, 1H, Ar-CH=CH), 4.68 (t, *J*=5.4 Hz, 1H, OH), 3.74 - 3,67 (m, 1H, NH-CH), 3.41 - 3.36 (m, 1H, CHH-OH), 3.33 - 3.27 (m, 1H, CHH-OH), 1.62 - 1.54 (m, 1H, CHH-CH₃), 1.38 - 1.28 (m, 1H, CHH-CH₃), 0.82 (d, *J*=7.5 Hz, 3H, CH₂-CH₃) |
| | | ¹³C NMR (126 MHz, DMSO-*d*₆) δ ppm 164.57 (C=O), 134.83 (Ar-CH=), 134.53 (Ar(4)), 133.04 (Ar(2)), 132.48 (Ar(3)), 129.94 (Ar(1)), 129.22 (Ar(6)), 128.58 (Ar(5)), 126.94 (Ar-CH=CH), 63.26 (CH₂-OH), 52.99 (NH-CH), 24.21 (CH₂-CH₃), 11.00 (CH₂-CH₃) |
| | | R_{M0}=3.08; logP (ChemSketch)= 2.62+/- 0.38; logP (ChemDraw)=2.92; ClogP (ChemDraw)=3.46 |
| 24 | **H-409/21** | R_{M0}=2.49; logP (ChemSketch)=2.16+/- 0.35; logP (ChemDraw)=2.29; ClogP (ChemDraw)=2.53 |
| 25 | **A-106** | M=267.75 [M+H]⁺ calculated 268.11, [M+H]⁺ found 268.19 (100%), Mp=115-117 °C |
| | | ¹H NMR (500 MHz, DMSO-*d*₆) δ ppm 7.75 (d, *J*=8.9 Hz, 1H, NH), 7.55 - 7.51 (m, 2H, Ar-H), 7.45 - 7.42 (m, 2H, Ar-H), 7.35 (d, *J*=16.0 Hz, 1H, Ar-CH=), 6.74 (d, *J*=15.8 Hz, 1H, Ar-CH=CH), 4.60 (t, *J*=5.4 Hz, 1H, OH), 3.70 - 3.64 (m, 1H, NH-CH), 3.43 - 3.35 (m, 2H, CH₂-OH), 1.88 - 1.80 (m, 1H, CH-(CH₃)₂), 0.86 - 0.79 (m, 6H, (CH₃)₂) |
| | | ¹³C NMR (126 MHz, DMSO-*d*₆) δ ppm 165.19 (C=O), 137.44 (Ar-CH=), 134.61 (Ar(4)), 134.20 (Ar(1)), 129.63 (2C, Ar(3,5)), 129.45 (2C, Ar(2,6)), 124.16 (Ar-CH=CH), 61.77 (CH₂-OH), 58.29 (NH-CH), 28.72 (CH-(CH₃)₂), 20.16 (CH-CH₃), 18.66 (CH-CH₃) |
| | | R_{M0}=2.76; logP (ChemSketch)=2.57+/- 0.37; logP (ChemDraw)=2.76; ClogP (ChemDraw)=3.14 |
| 26 | **A-99** | M=265.74 [M+H]⁺ calculated 266.09, [M+H]⁺ found 266.33 (100%), Mp=168-170 °C |
| | | ¹H NMR (500 MHz, DMSO-*d*₆) δ ppm 7.71 (d, *J*=8.6 Hz, 2H, Ar-H), 7.44-7.38 (m, 3H, Ar-H, Ar-CH=), 7.26 (d, *J*=15.5, 1H, Ar-CH=CH), 4.73 (d, *J*=4.0 Hz, 1H, OH), 3.99-3.92 (m, 2H, CHH (pip)), 3.72-3.64 (m, 1H, CH (pip)), 3.34-3.28 (m, 1H, CHH (pip)), 3.12-3.04 (m, 1H, CHH (pip)), 1.77-1.66 (m, 2H, CHH (pip)), 1.36-1.21 (m, 2H, CHH (pip)) |
| | | ¹³C NMR (126 MHz, DMSO-*d*₆) δ ppm 164.62 (C=O), 140.40 (Ar-CH=), 134.73 (Ar(C1)), 134.37 (Ar(C4)), 130.25 (2C, Ar(C2,C6), 129.25 (2C, Ar(C3,C5), 119.91 (Ar-CH=CH), 66.13 (CH-OH), 40.46 (CH₂-N)), 40.29 (CH₂-N), 35.64 (CH₂), 34.55 (CH₂) |
| | | R_{M0}=2.27; logP (ChemSketch)= 1.82+/-0.44; logP (ChemDraw)=1.74; ClogP (ChemDraw)=1.52 |
| | | physicochemical properties will be published soon |
| 27 | **H-310/20** | M=300.18 [M+H]⁺ calculated 300.06, [M+H]⁺ found 300.16 (100%), Mp=153-155 °C |
| | | ¹H NMR (500 MHz, DMSO-*d*₆) δ ppm 8.02 (d, *J*=8,6 Hz, 1H, Ar-H), 7.70 - 7.63 (m, 2H, Ar-H, Ar-CH=), 7.44 (dd, *J*=6.6, *J*=1.7 Hz, 1H, Ar-H), 7.33 (d, *J*= 15.5 Hz, 1H, Ar-CH=CH), 4.74 (d, *J*=4,3 Hz, 1H, OH), 3.98 - 3.91 (m, 3H, CHH (pip)), 3.72 - 3.66 (m, 1H, CH (pip)), 3.14 - 3.06 (m, 1H, CH (pip)), 1.77 - 1.67 (m, 2H, CHH (pip)), 1.36 - 1.22 (m, 2H, CHH (pip)) |
| | | ¹³C NMR (126 MHz, DMSO-*d*₆) δ ppm 164.15 (C=O), 135.57 (Ar-CH=), 134.98 (Ar(C4)), 134.62 (Ar(C2)), 132.46 (Ar(C3), 130.01 (Ar(C6)), 129.77 (Ar(C1), 128.26 (Ar(C5), 122.78 (Ar-CH=CH), 66.04 (CH-OH), 40.11 (CH₂-N)), 39.96 (CH₂-N), 35.58 (CH₂), 34.49 (CH₂) |
| | | R_{M0}=2.80; logP (ChemSketch)= 2.21+/- 0.46; logP (ChemDraw)=2.30; ClogP (ChemDraw)=2.23 |

### I. Biological activity of the compounds according to the invention

### Studies on the inhibition of the activity of commercial tyrosinase from mushroom (Sigma: T3824)

The following substrates were used for the enzymatic reaction: L-tyrosine and L-3,4-dihyroxyphenylalanine (L-DOPA). Both substrates were used in independent experiments. According to the literature, the use of L-DOPA enables the assessment of tyrosinase diphenolase activity, and L-tyrosine - the tyrosinase monophenolase activity. The determinations were performed in 96-well plates, in each well there was placed: 170 µL or 127.5 µL of 50 mM phosphate buffer (pH = 6.8) with tyrosinase (1U/well or 3U/well) and 10 µL of a solution of the tested compound in DMSO (the final concentration in the well was 500 µM, 250 µM or 125 µM). The negative control used 10 µL of pure solvent (DMSO). To initiate the reaction, an appropriate substrate was added: L-tyrosine (62.5 µL of solution in phosphate buffer) or L-DOPA (20 µL of solution in phosphate buffer). After a 25-minute incubation, the absorbance was measured at 475 nm using a microplate reader. The percentage of inhibition of tyrosinase activity was calculated using the formula: [(A_{C} -A_{T})/A_{C}] × 100, where A_{C} - is the absorbance of the negative control, A_{T} - is the absorbance of the sample containing the tested compound. The results are presented in **Table 3.**

### Cytotoxicity assessment in the B16-F10 murine melanoma cell line (ATTC CRL-6745) - preliminary safety assessment for subsequent studies

In order to determine safe doses of compounds for testing the inhibition of melanogenesis in a murine melanoma cell line, cytotoxicity tests were performed. Cytotoxicity was determined by the colorimetric method using MTT ([3-(4,5-dimethyl-2-thiazolyl)-2,5-diphenyl-2H-tetrazolium bromide, Sigma M5655). For this purpose, cells were transferred to 96-well plates in an amount of 5000/well. After 24 h of culture in an incubator in F 10-Ham medium with 1% FBS, the tested compounds at various concentrations were added to the wells together with fresh medium. DMSO was added to the medium in a negative control. The MTT test was performed after further culture for 48 h. Then the medium was removed and the formed formazan crystals were dissolved in DMSO. The absorbance was measured at 570 nm in a plate reader and the result was compared to the control (DMSO), which was 100%. The percentage of viability was calculated using the formula: [A_{T}/A_{C}^{∗}100], where A_{T} is the absorbance of the test sample, A_{C} - absorbance of the control sample. The results are presented in **Table 3.**

**Table 3. Results of inhibiting the activity of monophenolase (with L-DOPA) and diphenolase (with L-tyrosine) tyrosinase and cytotoxicity testing in B16F10 cells for the described compounds.**

| No. | Symbol | Tyrosinase inhibition with L-DOPA [%] | Tyrosinase inhibition with L-tyrosine [%] | Cytotoxicity study in B16F10 cells | | |
|---|---|---|---|---|---|---|
| | | | | Conc. [µM] % viability SD | | |
| 1 | **H-425/21** | 34.63±2.46% (500 µM) | 44.61±5.99% (500 µM) | 150 | 109.2 | 5.0 |
| | | | | 200 | 98.4 | 7.0 |
| | | | IC₅₀=763.70±50.34 µM | 250 | 86.9 | 6.9 |
| 2 | **H-426/21** | 30.66±1.82% (125 µM) | 39.89±3.21% (125 µM) | 150 | 30.3 | 4.0 |
| | | | | 200 | 28.2 | 5.4 |
| | | | | 250 | 28.3 | 5.5 |
| 3 | **A-133** | 47.96±1.83% (500 µM) | 58.56±4.58% (500 µM) | 150 | 101.7 | 5.0 |
| | | IC₅₀=522.94±27.29 µM | | 200 | 94.9 | 6.1 |
| | | | IC₅₀=449.52±35.45 µM | 250 | 95.6 | 5.6 |
| 4 | **A-111** | 59.94±1.56% (250 µM) | 88.98±0.98% (500 µM) | 150 | 88.2 | 4.2 |
| | | IC₅₀=146.71±16.82 µM | | 200 | 95.6 | 5.0 |
| | | | IC₅₀=36.98±1.07 µM | 250 | 89.7 | 3.3 |
| 5 | **H-297/20** | 20.53±2.84% (500 µM) | 19.09±2.53% (500 µM) | 150 | 102.3 | 7.6 |
| | | | | 200 | 73.6 | 8.8 |
| | | | | 250 | 88.5 | 6.7 |
| 6 | **H-415/21** | 30.43±0.88% (500 µM) | 25.04±5.67% (500 µM) | 150 | 85.2 | 7.1 |
| | | | | 200 | 90.3 | 7.4 |
| | | | | 250 | 75.7 | 5.9 |
| 7 | **A-43** | 34.55±4.18% (500 µM) | 35.50±1.37% (500 µM) | 150 | 108.9 | 10.6 |
| | | IC₅₀=1556.97±201.96 µM | | 200 | 105.7 | 4.4 |
| | | | IC₅₀=1032.93±48.26 µM | 250 | 103.2 | 7.6 |
| 8 | **H-331/21** | 64.31±0.21% (500 µM) | 82.50±0.87% (500 µM) | 12.5 | 49.0 | 9.0 |
| | | IC₅₀=152.87±14.0 µM | IC₅₀=103.40±1.95 µM | 25 | 47.8 | 8.3 |
| | | | | 50 | 54.2 | 10.3 |
| | | | | 150 | 32.7 | 3.1 |
| | | | | 200 | 51.9 | 4.7 |
| | | | | 250 | 52.7 | 3.1 |
| 9 | **H-298/21** | 11.16±2.78 (500 µM) | 20±2.96% (500 µM) | 150 | 46.5 | 3.3 |
| | | | | 200 | 40.6 | 5.5 |
| | | | | 250 | 37.4 | 5.2 |
| 10 | **H-411/21** | 16.89±7.94% (500 µM) | 20.43±2.09% (500 µM) | 150 | 50.6 | 6.0 |
| | | | | 200 | 34.9 | 7.4 |
| | | | | 250 | 41.2 | 6.6 |
| 11 | **KM-634** | 34.23±2.32% (500 µM) | 27.41±2.24% (500 µM) | 150 | 76.1 | 9.0 |
| | | | | 200 | 70.5 | 6.7 |
| | | | | 250 | 63.3 | 6.8 |
| 12 | **H-330/21** | 55.32±1.39% (500 µM) | 82.49±0.65% (500 µM) | 150 | 78.6 | 8.9 |
| | | IC₅₀=265.34±33.41 µM | | 200 | 82.9 | 8.9 |
| | | | IC₅₀=106.04±0.52 µM | 250 | 83.9 | 7.7 |
| 13 | **H-296/20** | 18.63±1.67% (500 µM) | 17.87±7.36% (500 µM) | 150 | 67.0 | 8.5 |
| | | | | 200 | 57.0 | 7.9 |
| | | | | 250 | 38.3 | 7.3 |
| 14 | **H-410/21** | 16.99±5.66% (500 µM) | 0.51±5.40% (500 µM) | 150 | 88.8 | 3.2 |
| | | | | 200 | 90.7 | 7.8 |
| | | | | 250 | 79.8 | 5.0 |
| 15 | **H-429/21** | 30.54±1.20% (500 µM) | 21.00±8.83% (500 µM) | 150 | 86.4 | 6.8 |
| | | | | 200 | 79.8 | 6.0 |
| | | | | 250 | 80.9 | 8.2 |
| 16 | **H-427/21** | 55.77±0.77% (500 µM) | 67.45±2.66% (500 µM) | 150 | 73.7 | 6.6 |
| | | IC₅₀=277.76±32.17 µM | | 200 | 80.2 | 4.4 |
| | | | IC₅₀=195.85±14.60 µM | 250 | 77.8 | 8.1 |
| 17 | **A-67** | 60.76±1.99% (500 µM) | 79.20±1.20% (500 µM) | 150 | 73.6 | 5.5 |
| | | IC₅₀=231.90±14.79 µM | | 200 | 87.8 | 6.2 |
| | | | IC₅₀=165.20±4.28 µM | 250 | 84.4 | 7.9 |
| 18 | **H-320/21** | 5.8±3.04% (500 µM) | 24.43±8.05% (500 µM) | 150 | 101.7 | 5.4 |
| | | | | 200 | 90.8 | 9.1 |
| | | | | 250 | 33.9 | 10.0 |
| 19 | **A-63** | 21.19±1.26% (500 µM) | 30.22±10.22% (500 µM) | 150 | 90.2 | 4.2 |
| | | | | 200 | 87.3 | 6.8 |
| | | | | 250 | 87.4 | 5.1 |
| 20 | **A-102** | 53.02±1.99% (500 µM) | 75.45±1.80% (500 µM) | 150 | 80.6 | 6.5 |
| | | IC₅₀=440.4±45.9 µM | | 200 | 83.5 | 7.6 |
| | | | IC₅₀=163.96±9.19 µM | 250 | 75.7 | 8.0 |
| 21 | **A-69** | 22.96±6.64% (500 µM) | 4.40±7.15% (500 µM) | 150 | 96.6 | 10.6 |
| | | | | 200 | 101.3 | 6.1 |
| | | | | 250 | 97.5 | 5.7 |
| 22 | **A-01** | 43.99±2.60% (500 µM) | 61.80±0.40% (500 µM) | 150 | 73.6 | 6.7 |
| | | IC50=734.09±38.93 | | 200 | 93.2 | 6.1 |
| | | µM | IC₅₀=284.45±4.49 µM | 250 | 85.4 | 6.7 |
| 23 | **H-285/20** | 0.21±5.64% (500 µM) | 14.56±1.63% (500 µM) | 150 | 84.2 | 6.2 |
| | | | | 200 | 74.8 | 8.0 |
| | | | | 250 | 24.6 | 5.7 |
| 24 | **H-409/21** | 21.81±2.31% (500 µM) | 15.31±8.27% (500 µM) | 150 | 83.2 | 6.9 |
| | | | | 200 | 95.3 | 5.3 |
| | | | | 250 | 103.4 | 5.0 |
| 25 | **A-106** | 41.17±0.81% (500 µM) | 57.62±2.11% (500 µM) | 150 | 85.9 | 7.7 |
| | | IC₅₀=905.77±81.10 µM | | 200 | 88.1 | 8.8 |
| | | | IC₅₀=405.22±8.44 µM | 250 | 72.6 | 5.7 |
| 26 | **A-99** | 54.87±1.47% | 75.14±1.60% (500 µM) | 150 | 109.8 | 11.1 |
| | | IC₅₀=450.29±20.81 µM | | 200 | 102.6 | 10.4 |
| | | | IC₅₀=176.89±5.37 µM | 250 | 96.6 | 10.5 |
| 27 | **H-310/20** | 7.23±0.81% (500 µM) | 19.24±3.44% (500 µM) | 150 | 89.5 | 4.7 |
| | | | | 200 | 100.4 | 12.7 |
| | | | | 250 | 93.0 | 6.8 |

### Studies on the kinetics of the enzymatic reaction catalyzed by tyrosinase.

Various concentrations of the substrate (L-DOPA or L-tyrosine) and the tested compounds were used to study the kinetics of inhibition of the enzymatic reaction catalyzed by tyrosinase with L-DOPA or L-tyrosine. The tests were carried out on 96-well plates using a microplate reader capable of measuring the increase in absorbance over time. Lineweaver-Burk graphs were prepared from the obtained results and the type of inhibition was determined based on the graph layout.

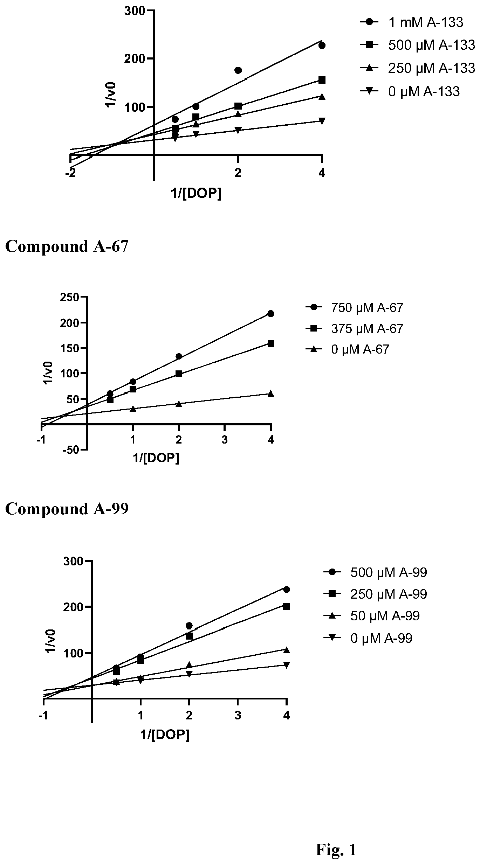

Studies on the kinetics of tyrosinase inhibition showed that the compounds are characterized by a mixed type of inhibition. This is indicated by the intersection of the graphs of the relationship between the reciprocal of the rate of the initial enzymatic reaction and the reciprocal of the concentration of the substrate (L-tyrosine or L-DOPA) for various concentrations of the tested inhibitor. As an example, Lineweaver-Burk plots are presented for compounds **A-111, H-330/21, A-133, A-67** and **A-99** (Fig. 1).

Fig. 1 shows Lineweaver-Burk plots for various concentrations of the tested compounds - the relationship between the reciprocal of the rate of the initial enzymatic reaction and the reciprocal of the concentration of the reaction substrate (L-DOPA or L-tyrosine).

### Inhibition of melanin synthesis in B16F10 murine melanoma cells

Only those concentrations of compounds at which they did not show cytotoxicity (viability in the MTT test >80%) were selected for testing the inhibition of melanogenesis in B-16-F10 cells.

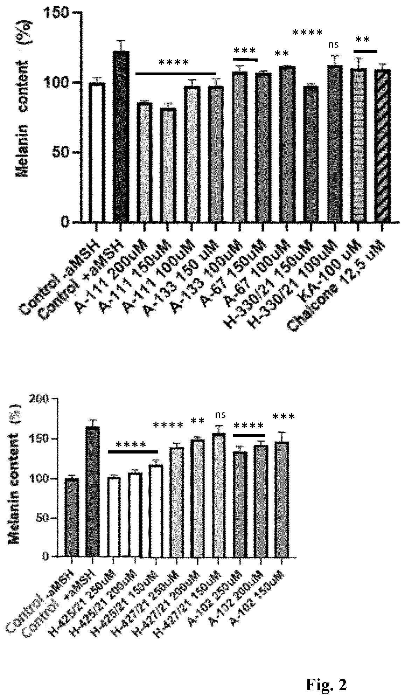

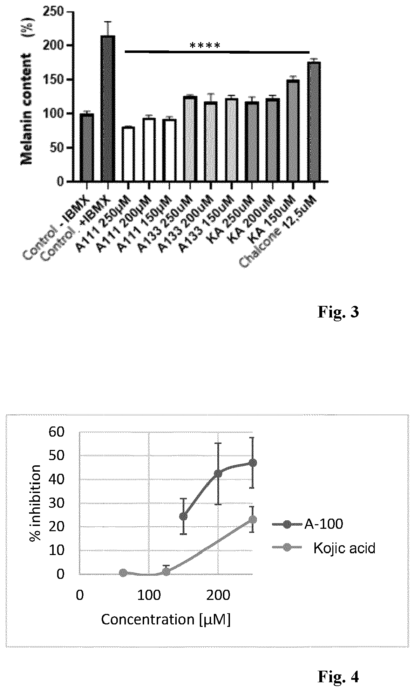

For this purpose, 50,000 cells were placed in each well of a 24-well plate (in F10-Ham medium, 1% FBS). After 24 h of culture, the tested compounds were added in fresh medium (DMEM, 1% FBS), with additional 5 µM α-melanocorticotropic hormone (α-MSH) or 100 µM 3-isobutyl-1-methylxanthine (IBMX) in the medium in order to induce melanogenesis. On each plate, 3 wells constituted a control sample without the melanogenesis inducer (α-MSH) and without the tested compounds. After 48 hours of incubation under these conditions, the medium was removed and the cells were washed twice with PBS. Then, 150 µL of 1M NaOH was added to each well and heated for 1 hour at 60 °C. Then, 100 µL of the well contents were transferred to a 96-well plate and the absorbance was measured at 405 nm in a microplate reader. The absorbance for the control sample without α-MSH was assigned a value of 100%. The results of inhibition of melanin synthesis based on the melanin content in cells induced with α-MSH or IBMX and the tested compounds are presented below (**Fig. 2****,** **Fig. 3**).

Fig. 2 shows the inhibition of α-melanocorticotropic hormone-induced melanogenesis in B16-F10 cells (p<0.001 (****), 0.001<p<0.05 (***), p<0.05 (^{∗∗}) , ns - no statistical significance, ANOVA/Bonferroni test).

Fig. 3 shows the inhibition of 3-isobutyl-1-methylxanthine-induced melanogenesis in B16-F10 cells (p<0.001, ANOVA/Bonferroni test).

### Inhibition of tyrosinase activity released from B16-F10 cells

50,000 cells were placed in each well of a 24-well plate (in F10-Ham medium, 1% FBS). After 24 h of culture, the tested compounds were added in fresh medium (DMEM, 1% FBS), with an additional 5 µM of α-melanocorticotropic hormone (α-MSH) in the medium to induce melanogenesis. Three wells on the plate constituted a control sample without the melanogenesis inducer (α-MSH) and without the tested compounds. After 24 hours of incubation under these conditions, the medium was removed and the cells were washed twice with PBS. 100 µL of lysis buffer composed of 90 µL of 50 mM potassium phosphate buffer, 5 µL of 0.1 mM phenylmethylsulfonyl fluoride and 5 µL of 1% Triton X-100 were added to the wells. The plates were placed at -80 °C for 30 minutes. Cell lysates were then centrifuged for 30 min (4 °C, 12,000 rpm), and then 80 µL of the supernatant was transferred to 96-well plates. To initiate the enzymatic reaction, 20 µL of L-DOPA was added so that the final concentration was 0.5 mM. After a 30-minute incubation, the absorbance was measured at 475 nm using a microplate reader. The percentage of inhibition of tyrosinase activity was calculated using the formula: [(A_{C} -A_{T})/A_{C}] × 100, where A_{C} - is the absorbance of the negative control (from wells in which cells were cultured without the tested compounds), A_{T} - is the absorbance of the sample from wells in which cells were cultured in the presence of the test compound. The obtained results are presented in **Fig. 4****.** Compound **A-111** was shown to inhibit the activity of tyrosinase released from B16-F10 cells.

**Fig. 4** shows the inhibition of free tyrosinase from B16-F10 cells.

### Skin irritation tests in a reconstructed human epidermis model (Episkin^{™})

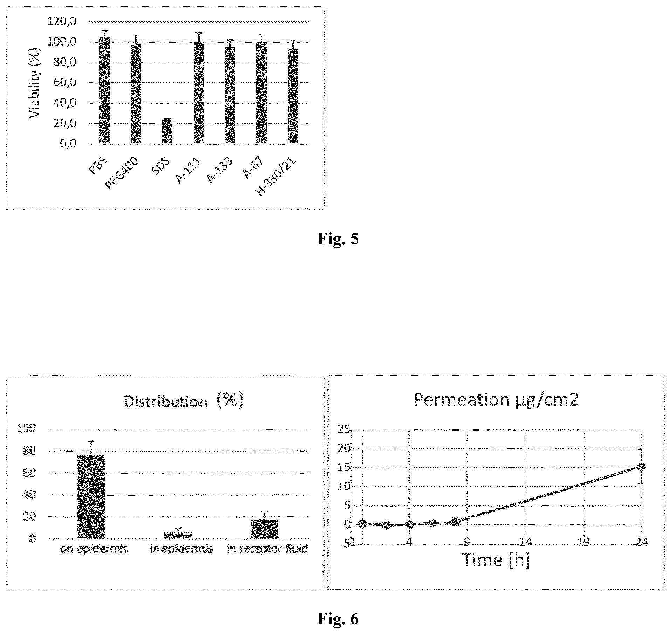

The study used human reconstructed epidermis Episkin^{™} with an area of 1.07 cm². Immediately after receipt, the tissue was transferred to fresh medium and cultured in an incubator (37 °C, 5% CO₂) for 18 hours. Before performing experiments, the inserts were washed with sterile PBS and placed in the wells of a 12-well plate containing 2 mL of fresh culture medium. Then, the tested substances **A-111, A-133, A-67** and **H-330/21** were applied onto the epidermis in the form of solutions in PEG400 in an amount of 26 mg of solution/cm². After 15 min of exposure, samples were washed with PBS (5x5 mL). The inserts were transferred to plates with fresh culture medium and incubated for 42 hours (37 °C, 5% CO₂). After this time, the entire medium was collected to assess the amount of released interleukin 1α (the medium was stored at -20 °C until the experiment was performed), and the inserts were transferred to the MTT solution. After 3 hours of incubation in MTT solution, a dark purple coloration of the tissue was observed, the epidermis was separated from the collagen matrix, and the formed formazan was extracted into acidified isopropanol. Measurement of the absorbance of the samples obtained in this way at 570 nm enabled the assessment of tissue viability according to the formula: % Viability = [OD (570 nm) for the tested compound/OD (570 nm) for the control] × 100. The obtained results of viability determination (**Fig. 5**) and IL-1α level in the culture medium (**Table 4**) indicates the lack of irritating effect of the tested compounds. The positive control, i.e. 5% aqueous sodium lauryl sulfate (SDS), reduced tissue viability to 23.8% compared to the negative control (averaged result for PBS and PEG400) and caused a significant increase in IL-1α release.

**Fig. 5** shows the results of the skin irritation test in a model of reconstructed human epidermis.

**Table 4. Results of measuring the concentration of interleukin 1α in the culture medium in the skin irritation test in a model of reconstructed human epidermis.**

| Tested sample | IL-1α concentration in the culture medium [pg/mL] [pg/mL] |
|---|---|
| PBS | 9.89 ± 4.75 |
| SDS | > 250 |
| PEG | 16.20 ± 8.19 |
| A-111 | 20.70 ± 10.96 |
| A-133 | 26.45 ± 16.32 |
| A-67 | 9.83 ± 1.69 |

### Skin permeation studies in a reconstructed human epidermis model (Episkin^{™})

The study used human reconstructed epidermis Episkin^{™} with an area of 1.07 cm². Immediately after receipt, the tissue was transferred to fresh medium and cultured in an incubator (37 °C, 5% CO₂) for 18 hours. Before performing the experiments, the inserts were washed with sterile PBS and placed in the 12-well plate containing 2 mL of receptor fluid (PBS+0.25% Tween 20). The tested substance **A-111** in a macrogol formulation (2% **A-111** in the PEG400+PEG800 base) was applied onto the epidermis in an amount of approximately 10 mg/cm² ("finite dose application") using a sterile inoculation loop. The amount applied was weighed accurately. As a control, a macrogol formulation without the test substance was applied onto the epidermis. The finished experimental plate was incubated at 32 °C. At specific time intervals, aliquots of the receptor fluid (0.5 mL) were collected for the analysis and replaced with 0.5 mL of fresh receptor fluid. After 24 h of culture, all of the receptor fluid was collected. The examined tissue was washed with cotton buds soaked in methanol. The donor and receptor compartments were washed with methanol, and the epidermis was separated from the inserts and transferred to methanol. Samples were stored at -20 °C until analysis. The content (concentration) of the tested substance **A-111** in the receptor fluid and in individual compartments: on the surface of the epidermis, in the epidermis, in the receptor fluid was determined using measurements in a microplate reader at 281 nm on 96-well plates transmitting ultraviolet radiation. The study showed that compound **A-111** crosses the epidermal barrier and has the potential to penetrate the dermis. The obtained results indicate that there is no accumulation of compound **A-111** in the epidermis (**Fig. 6**).

**Fig. 6** shows the results of an epidermal permeation study of compound **A-111** using reconstructed human epidermis Episkin^{™}.

### Melanogenesis inhibition studies in a model of reconstructed human epidermis containing melanocytes (MelanoDerm by Mattek).

The research was carried out on reconstructed human epidermis containing melanocytes MelanoDerm^{™} MEL-300-B from MatTek (Bratislava, Slovakia). The ratio of keratinocytes to melanocytes in the epidermis used is 10:1. Only medium purchased from the same manufacturer (EPI-100-NMM-113) was used to culture the epidermis during the experiments. Once the epidermis was obtained, it was transferred to 12-well plates containing 5 mL of medium/well. Plates were used to enable proper contact of the lower parts of the inserts with the substrate (so-called hanging-top plate). Solutions of the compounds were prepared immediately before their application. The solutions were applied to the inserts using a positive displacement pipette and then distributed using a sterile glass bulb headed Pasteur pipette. The application was repeated every 48 hours, a total of 7 applications were performed. Before the next application, each insert was rinsed with sterile DPBS and dried with a sterile cotton buds. The application was also accompanied by a change of the medium. All work during epidermis culture was carried out in a laminar air flow chamber, using aseptic principles and sterile reagents and utensils. The epidermis was cultured in an incubator ensuring appropriate humidity, temperature (37 °C) and CO₂ concentration (5%).

The experiment was conducted for 14 days. After this time, the inserts were rinsed with DPBS and dried with a cotton swab. For the selected pool of inserts, tissue viability was assessed using the MTT (Thiazolyl Blue Tetrazolium bromide) test, the remaining ones were used to determine the amount of melanin. To assess viability, the inserts were transferred to 24-well plate containing 0.3 mL of DMEM medium with 1 mg/mL MTT and incubated for 3 hours in an incubator. The epidermis was then placed in 2 mL of isopropanol and the resulting formazan was extracted for 2 hours at room temperature. The absorbance of the samples was measured at 570 nm in a microplate reader and correlated with the absorbance of the control by the formula: % Viability = [OD (570 nm) for test compound/OD (570 nm) for control] × 100. To determine the amount of melanin, the inserts were incubated in 5% sodium bicarbonate for 30 min, then dried and placed in 0.5 mL of Solvable^{™} reagent, which caused complete lysis of the epidermis. After heating at 95 °C, the absorbance of the samples was measured at 405 nm in a microplate reader. On this basis, the reduction in the amount of melanin was calculated in relation to the control sample. The absorbance results were also correlated with a standard curve for synthetic melanin. Table 5 shows the viability and melanin content results calculated from absorbance measurements.

**Table 5. Viability and melanin content in the MelanoDerm epidermis after application of the tested samples, calculated on the basis of absorbance measurements.**

| **Tested sample** | | **Melanin content [% control, mean ± SD]** | **Viability [% control, mean ± SD]** |
|---|---|---|---|
| **Water** (25 µL) | | 100.00±3.27 | 100.00±3.46 |
| **50% PEG** (25 µL) | | 97.25±5.23 | 95.33±5.70 |
| **A-111** | 1.0% in 50% PEG (10 µL) | 94.95±3.57 | 101.45±6.45 |
| | 1.0% in 50% PEG (25 µL) | 81.23±6.53 | 85.67±7.25 |
| | 2.0% in 50% PEG (25 µL) | 67.26±9.18 | 106.64±7.78 |
| **A-133** | 1.0% in 50% PEG (10 µL) | 98.62±9.47 | 101.22±6.52 |
| **H-425/21** | 1.0% in 50% PEG (10 µL) | 102.98±4.25 | 87.16±6.30 |

### Extended skin irritation tests on reconstructed human epidermis - exposure time extended to 7 days.

In order to perform extended skin irritation (safety) tests on the reconstructed human epidermis, the exposure time to the tested compounds was extended to 2 and 7 days in the human reconstructed epidermis model EpiDerm^{™} from MatTek. The epidermis was cultured according to the manufacturer's recommendations, using appropriate multi-well plates and culture media. Solutions of the tested compounds were applied to the epidermis using a positive displacement pipette and distributed using a sterile glass bulb headed Pasteur pipette. All work related to the application of the tested substances was carried out in a laminar air flow chamber, using aseptic principles and sterile reagents and utensils. The epidermis was cultured in an incubator ensuring appropriate humidity, temperature (37 °C) and CO₂ concentration (5%). The medium was replaced daily. After an appropriate incubation time, i.e. 2 days or 7 days, the epidermis was rinsed with sterile DPBS and incubated with MTT (Thiazolyl Blue Tetrazolium bromide) solution at 37 °C for 3 hours. After this time, a purple color of formazan was observed, correlated with tissue viability. The resulting formazan was extracted into isopropanol, then the absorbance was measured at 570 nm and the tissue viability relative to the control was calculated according to the formula: %Viability = [OD (570 nm) for the test compound/OD (570 nm) for the control] × 100. Moreover, in the medium collected after the first 24 hours of EpiDerm epidermis culture, the level of interleukin 1α (Il-1α), which is a mediator of inflammation, was determined. Its level correlates with the unfavorable (irritating) effect of the tested substances on the epidermis. The level of IL-1α was determined using the Cayman Elisa test. Table 6 shows the viability results and the results of measuring the concentration of interleukin 1α in the culture medium in the irritation study in a reconstructed human epidermis model.

**Table 6. Viability results and results of interleukin 1α concentration measurement in the culture medium in the irritation test in the reconstructed human epidermis model EpiDerm.**

| **Tested sample** | | **Viability [% of control, mean±SD]** | | **Il-1α in culture medium [pg/mL]** |
|---|---|---|---|---|
| | | **2 days** | **7 days** | |
| **50% PEG** | 10 µL | 100.00±0.52 | 100.00±6.94 | 15.89±0.95 |
| **A-111** | 0.1% in 50% PEG (10 µL) | 88.42±1.25 | 107.77±3.12 | - |
| | 0.5% in 50% PEG (10 µL) | | | |
| | 1.0% in 50% PEG (10 µL) | 92.02±3.47 | 113.85±4.03 | - |
| | | 98.56±11.78 | 100.41±1.52 | 15.89±.057 |
| **A-133** | 1.0% in 50% PEG (10 µL) | 107.19±7.45 | 107.26±21.82 | 34.99±1.17 |
| **H-425/21** | 1.0% in 50% PEG (10 µL) | 99.75±15.49 | 104.01±1.11 | 60.29±1.08 |

### Extended skin permeation studies in reconstructed human epidermis (confirmation of bioavailability).

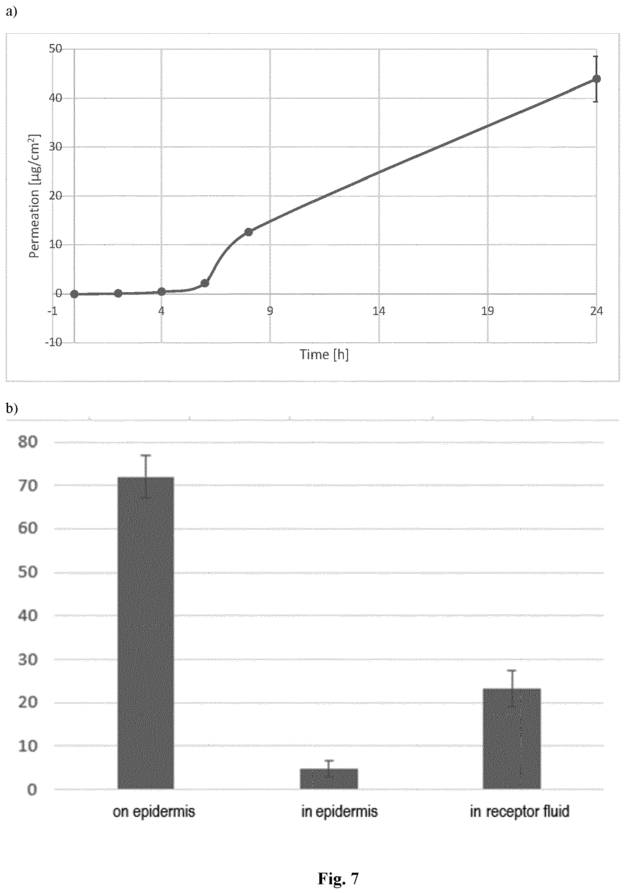

In order to confirm proper bioavailability, studies of the permeation of compound **A-111** from a solution in PEG400 were performed in a model of reconstructed human epidermis. Human reconstructed epidermis Episkin^{™} with an area of 1.07 cm² (Episkin, Lyon, France) was used for this purpose. Immediately after receipt, the tissue was transferred to fresh medium and cultured in an incubator (37 °C, 5% CO₂) for 18 hours. Before performing the experiments, the inserts were washed with sterile PBS and placed in the 12-well plate containing 2 mL of receptor medium (PBS+0.25% Tween 20). The tested substance **A-111** was applied onto the epidermis in the form of a 2% PEG400 solution in an amount of 15 µL. The finished experimental plate was incubated at 32 °C. At specific time intervals, aliquots of the receptor fluid (0.5 mL) were collected for the analysis and replaced with fresh fluid. After 24 h of culture, all of the receptor fluid was collected. The examined tissue was washed with cotton buds soaked in methanol. The donor and receptor compartments were washed with methanol, and the epidermis was separated from the inserts and transferred to methanol. The content (concentration) of the tested substance **A-111** in the acceptor fluid and in individual compartments: on the surface of the epidermis, in the epidermis, in the receptor fluid was determined using measurements in a microplate reader at 281nm on 96-well plates transmitting ultraviolet radiation. The study showed that compound **A-111** crosses the epidermal barrier and has the potential to penetrate the dermis. Fig. 7 shows: (a) penetration through the epidermis of **A-111** from a 2% solution in PEG400 and (b) decomposition [%] of the compound **A-111** in reconstructed human epidermis after 24 hours from application in a 2% PEG400 solution (UV analysis).

### Safety studies

### a) in human normal cells: keratinocytes, fibroblasts, primary melanocytes, in human cancer cells: HepG2, SH-SY5Y

In order to test the safety of the compounds, both normal human cells were used: keratinocytes (HaCaT), fibroblasts (BJ), primary melanocytes that occur in the skin, as well as human cancer cells: the hepatocarcinoma line HepG2 and the neuroblastoma line SH-SY5Y, which are considered a model for the assessment of hepatocytotoxicity and neurocytotoxicity. The concentrations of the tested compounds in the range of 250-100 µM were used in the studies. The cell lines used in the research were purchased from the American Type Culture Collection (ATCC) and were cultured in media recommended by the manufacturer with the addition of FBS and antibiotics in an incubator ensuring appropriate culture conditions, i.e. humidity, temperature and CO₂ concentration. Cells were seeded into multiwell plates and incubated with the test compounds for 48 hours. After this time, MTT and LDH tests were performed to assess cell viability. The MTT test is based on the ability to transform the substrate: [3-(4,5-dimethyl-2-thiazolyl)-2,5-diphenyl-2H-tetrazolium bromide into a colored product only by living cells. The absorbance at 570 nm, which corresponds to the absorption maximum for the product, is directly proportional to cell viability. The LDH test is based on testing the activity of the enzyme lactate dehydrogenase in the culture medium, which is released from cells as a result of damage to the cell membrane, e.g. as a result of cell death. Triton X-100 was used as a positive control in the test. The determination is colorimetric, the substrate is transformed by the released enzyme into a colored product, an increase in absorbance indicates a larger amount of the released enzyme, and thus confirms greater damage to cell membranes. Tables 7 and 8 present the results obtained, which correspond to the viability of cells incubated with the compounds in relation to the viability of cells constituting a control sample, i.e. incubated with the solvent (DMSO) at the same concentration as in the case of the groups tested in the MTT test or in relation to the results obtained for the Triton X-100 positive control in the LDH test. They indicate that the compounds are safe at the concentrations tested.

**Table 7. Viability of tested normal human cell lines: keratinocytes (HaCaT), fibroblasts (BJ) and primary melanocytes after incubation with the tested compounds - determination in the MTT test (% of solvent control ± SD) and LDH (percentage of positive control - Triton X-100.**

| **Tested compound** | **Concentration [µM]** | **Keratinocytes (HaCaT)** | | **Fibroblasts (BJ)** | | **Primary melanocytes** | |
|---|---|---|---|---|---|---|---|
| | | **MTT** | **LDH** | **MTT** | **LDH** | **MTT** | **LDH** |
| **H-330/21** | 250 | 94.89±1.06 | 24.24 | 76.51±1.80 | 28.74 | 90.27±2.25 | 18.10 |
| | 200 | 97.07±2.81 | 17.03 | 85.52±3.08 | 14.25 | 93.78±2.29 | 10.91 |
| | 150 | 101.42±2.04 | 5.33 | 94.85±3.51 | 6.50 | 97.02±4.49 | 13.09 |
| | 100 | 102.03±1.45 | 3.66 | 100.54±5.01 | 4.74 | 101.42±2.21 | 5.63 |
| **A-133** | 250 | 95.53±2.80 | 19.58 | 82.50±3.99 | 28.31 | 88.88±2.26 | 19.45 |
| | 200 | 101.51±2.49 | 6.38 | 87.76±2.71 | 14.72 | 97.05±2.96 | 16.71 |
| | 150 | 101.40±2.08 | 3.08 | 91.68±3.64 | 4.47 | 101.88±2.47 | 7.59 |
| | 100 | 101.35±2.17 | 3.23 | 99.41±5.96 | 5.00 | 103.69±2.57 | 4.68 |
| **A-111** | 250 | 91.57±2.10 | 13.55 | 94.51±5.03 | 14.90 | 93.39±1.60 | 18.41 |
| | 200 | 101.04±2.77 | 6.49 | 98.94±4.04 | 8.43 | 99.25±1.92 | 13.38 |
| | 150 | 101.56±1.94 | 4.43 | 100.94±3.37 | 5.37 | 103.09±1.79 | 4.95 |
| | 100 | 101.68±3.45 | 4.69 | 99.88±5.25 | 5.25 | 104.51±1.19 | 3.31 |
| **H-427/21** | 250 | 89.94±4.66 | 17.79 | 98.13±2.81 | 17.32 | 85.19±2.03 | 15.01 |
| | 200 | 99.24±3.00 | 6.50 | 100.37±3.92 | 9.00 | 93.78±2.18 | 4.31 |
| | 150 | 98.15±2.63 | 3.69 | 100.76±3.34 | 5.92 | 100.57±2.18 | 4.44 |
| | 100 | 100.47±3.65 | 2.76 | 101.82±3.99 | 2.75 | 102.20±2.24 | 3.93 |
| **H-415/21** | 250 | 88.54±3.74 | 18.19 | 92.86±4.30 | 16.63 | 87.64±1.44 | 14.94 |
| | 200 | 100.88±1.40 | 10.18 | 96.13±3.64 | 9.82 | 94.81±2.92 | 8.34 |
| | 150 | 101.78±2.32 | 6.70 | 101.92±2.88 | 8.75 | 99.89±2.43 | 6.01 |
| | 100 | 101.11±2.03 | 2.95 | 102.12±2.71 | 8.81 | 103.27±1.88 | 4.22 |

**Table 8. Viability of the tested human cancer cell lines: hepatocarcinoma HepG2 and neuroblastoma SH-SY5Y after incubation with the tested compounds - determination in the MTT test (% of solvent control ± SD) and LDH (percentage of positive control - Triton X-100.**

| **Tested compound** | **Concentration [µM]** | **Hepatocarcinoma HepG2** | | **Neuroblastoma SH-SYSY** | |
|---|---|---|---|---|---|
| | | **MTT** | **LDH** | **MTT** | **LDH** |
| **H-330/21** | 250 | 93.16±1.71 | 19.02 | 99.11±3.66 | 13.41 |
| | 200 | 100.86±6.41 | 12.16 | 99.79±3.97 | 8.35 |
| | 150 | 100.44±3.93 | 4.90 | 99.86±3.76 | 4.90 |
| | 100 | 100.73±4.51 | 5.38 | 99.79±5.34 | 6.19 |
| **A-133** | 250 | 98.92±2.34 | 16.13 | 100.41±1.87 | 13.32 |
| | 200 | 99.09±1.60 | 9.65 | 100.62±5.99 | 8.49 |
| | 150 | 100.66±7.39 | 7.07 | 100.75±3.01 | 6.93 |
| | 100 | 104.96±7.60 | 5.31 | 101.50±3.33 | 5.33 |
| **A-111** | 250 | 96.27±2.57 | 9.75 | 99.93±4.36 | 6.77 |
| | 200 | 98.19±3.27 | 5.48 | 100.55±2.31 | 5.98 |
| | 150 | 97.87±3.27 | 4.73 | 99.32±3.36 | 4.61 |
| | 100 | 101.10±6.01 | 4.90 | 100.14±3.22 | 4.90 |
| **H-427/21** | 250 | 100.08±10.58 | 13.51 | 102.60±9.66 | 8.87 |
| | 200 | 97.07±4.00 | 5.98 | 101.30±2.94 | 5.98 |
| | 150 | 98.44±3.94 | 4.45 | 100.96±2.72 | 4.45 |
| | 100 | 99.61±4.37 | 3.09 | 100.48±3.67 | 3.09 |
| **H-415/21** | 250 | 98.90±4.21 | 15.47 | 101.71±2.38 | 10.55 |
| | 200 | 100.53±3.98 | 8.92 | 100.27±3.50 | 8.92 |
| | 150 | 99.54±5.74 | 7.24 | 99.32±2.96 | 7.24 |
| | 100 | 100.32±1.75 | 3.90 | 100.68±4.63 | 3.90 |

### b) Ames test

The study used the Ames microplate test (Ames MPF^{™} Penta 2 by Xenometrix, Allschwil, Switzerland). Growth medium, exposure medium, indicator medium, positive controls, hepatic microsomal fraction, NADP, glucose-6-phosphate, *Salmonella typhimurium* and *Escherichia coli* strains were components of the purchased experimental kit. Bacteria were grown overnight in growth medium. They were then exposed to the test compound at the appropriate concentration in exposure medium without or with the rat microsomal fraction S9 in 24-well plates for 90 min at 37 °C with shaking. Then, indicator medium was added to the wells and pipetted into 384-well plates, which were incubated for 48 hours at 37 °C without shaking. Results were assessed by counting the wells in which the medium turned yellow for each experimental condition.

Table 9 contains the results of the experiments performed in the form of a parameter of increasing the number of yellow wells on 384-well plates compared to the reference level (reference level - average number of discolored wells for the solvent + standard deviation). None of the tested compounds at any concentration caused a greater than 2-fold increase in the index compared to the value for the control sample (i.e. samples with only solvent at the same concentration as in the tested samples). For compounds **A-111, A-133, H-425/21** and **A-67,** the same conclusions can be drawn from experiments that include samples and in which metabolic activation was used (rat liver microsomal fraction S9). To sum up, no mutagenic properties of the tested compounds were observed, both in terms of base substitution and frameshift mutation.

**Table 9. Ames microplate test results.**

| **Cmpd** | **Cone. [mM]** | **Increase in the number of yellow wells compared to the reference level*** | | | | |
|---|---|---|---|---|---|---|
| | | ***S. typhimurium*** | | | | ***E. coli*** |
| | | **TA98** | **TA100** | **TA1535** | **TA1537** | **WP2** |
| **A-111** | 0.5 | 0.7 | 0.74 | 0.55 | 0.80 | 1.46 |
| | 1.0 | 0.44 | 0.39 | 0.18 | 0.54 | 1.10 |
| | 2.0 | 1.00 | 0.42 | 1.65 | 0.54 | 1.10 |
| | PC** | 16.00 | 5.10 | 26.35 | 38.58 | 52.71 |
| **A-111 +S9** | 0.5 | 1.50 | 0.97 | 0.58 | 0.27 | 0.62 |
| | 1.0 | 0.83 | 0.47 | 0.35 | 0.45 | 0.71 |
| | 2.0 | 1.50 | 0.67 | 0.23 | 0.27 | 0.98 |
| | PC** | 24.00 | 4.00 | 10.25 | 11.34 | 9.52 |
| **A-133** | 0.5 | 0.44 | 0.60 | 0.92 | 1.07 | 0.73 |
| | 1.0 | 0.33 | 1.03 | 0.55 | 0.54 | 0.37 |
| | 2.0 | 0.67 | 0.57 | 1.10 | 0.54 | 1.10 |
| | PC** | 16.00 | 5.10 | 26.35 | 38.58 | 52.71 |
| **A-133 +S9** | 0.5 | 0.50 | 0.83 | 0.47 | 0.18 | 0.27 |
| | 1.0 | 0.67 | 0.78 | 0.23 | 0.36 | 0.44 |
| | 2.0 | 1.00 | 0.61 | 0.35 | 0.27 | 0.53 |
| | PC** | 24.00 | 4.00 | 10.25 | 11.34 | 9.52 |
| **H-425/21** | 0.5 | 0.27 | 0.73 | 1.04 | 0.09 | 0.17 |
| | 1.0 | 0.54 | 0.80 | 0.59 | 0.18 | 0.67 |
| | 2.0 | 0.80 | 0.73 | 0.45 | 0.82 | 1.00 |
| | PC** | 38.32 | 9.40 | 21.39 | 13.18 | 23.83 |
| **H-425/21 +S9** | 0.5 | 0.42 | 0.50 | 0.54 | 1.33 | 0.75 |
| | 1.0 | 0.42 | 0.69 | 0.40 | 0.00 | 0.83 |
| | 2.0 | 0.63 | 0.58 | 0.94 | 1.00 | 0.75 |
| | PC** | 15.03 | 4.00 | 15.41 | 39.33 | 8.00 |
| **A-67** | 0.5 | 0.27 | 1.53 | 0.30 | 0.00 | 0.50 |
| | 1.0 | 1.07 | 0.93 | 0.45 | 0.18 | 1.00 |
| | 2.0 | 1.07 | 0.80 | 1.04 | 0.82 | 0.83 |
| | PC** | 38.32 | 9.40 | 21.39 | 13.18 | 23.83 |
| **A-67 +S9** | 0.5 | 0.31 | 0.78 | 0.27 | 1.00 | 0.83 |
| | 1.0 | 0.94 | 0.64 | 0.54 | 1.33 | 0.42 |
| | 2.0 | 1.04 | 0.31 | 0.13 | 0.00 | 1.42 |
| | PC** | 15.03 | 4.00 | 15.41 | 39.33 | 8.00 |
| **A-102** | 0.5 | 0.24 | 1.08 | 0.55 | 0.33 | 0.48 |
| | 1.0 | 0.24 | 0.75 | 0.14 | 0.00 | 1.03 |
| | 2.0 | 0.35 | 0.92 | 0.89 | 1.00 | 0.79 |
| | PC** | 17.01 | 12.00 | 9.90 | 48.00 | 11.43 |
| **H-415** | 0.5 | 0.47 | 0.92 | 0.21 | 0.33 | 0.64 |
| | 1.0 | 0.24 | 0.58 | 0.41 | 0.33 | 0.40 |
| | 2.0 | 0.35 | 0.75 | 0.10 | 0.00 | 0.95 |
| | PC** | 17.01 | 12.00 | 9.90 | 48.00 | 11.44 |

| | | | | | | |
|---|---|---|---|---|---|---|
| reference level = solvent mean + 1 SD; SD - standard deviation; **Positive controls: 2-nitrofluorene (2-NF) 2 µg/mL (TA98), 4-nitoquinolinc-*N*-oxide (4-NQO) 0.1 µg/mL (TA100), N4-aminocytidine (*N4-ACT*) 100 µg/mL (TA1535), 9-aminoacridine (9-AAc) 15 µg/mL (TA1537), 4-NQO 2 µg/mL (*E. coli* uvrA[pKM101]), 2-aminoanthracene (2-AA) 0.5 µg/mL (TA98, + S9); 2-AA 125 µg/mL (TA100, + S9); 9-AA at 2.5 µg/mL (TA1535 and TA1537, + S9); 2-aminofluorene (2-AF) at 400 µg/mL (*E.coli* uvrA[pKM101], + S9). | | | | | | |

### c) genotoxicity in the micronucleus test

The assay was performed using a Chinese hamster ovary cell line (CHO-K1) purchased from the American Type Culture Collection (ATCC). The cells were cultured in F-12K medium with the addition of 10% fetal bovine serum (FBS) and antibiotics (streptomycin/penicillin) in an incubator at 37 °C and 5% CO₂ concentration). Before the actual micronucleus test, a cytotoxicity assessment was performed to ensure that the actual test was performed only at safe concentrations. For this purpose, cells were transferred to 96-well plates and incubated with the tested compounds for 24 hours. After this time, the MTT test was performed and cell viability was assessed in comparison to the control group (cells cultured with DMSO at the same concentration as in the case of the tested samples). Table 10 presents the results of the cytotoxicity assessment of the tested compounds.

For the micronucleus assay, cells were transferred to 25 cm² culture vessels at 10⁶/vessel and incubated for 24 hours (37 °C, 5% CO₂). Then, the culture medium was replaced with fresh one containing the tested compounds, solvent as a negative control or mitomycin (0.5 µg/mL) as a positive control. After 24 hours of incubation, the cells were washed twice with PBS, and culture medium with cytochalasin B at a concentration of 4.5 µg/mL was added to the vessels to inhibit mitotic divisions. The cells were cultured for another 24 hours. After this time, the cells were trypsinized and microscopic preparations were prepared from them (after washing with sodium citrate and fixing with methanol, acetic acid and formaldehyde). Cells on slides were stained with Giemsa stain. Then, microscopic analysis of the preparations was performed using an inverted microscope with a 40-fold magnifying objective (Leica Microsystems GmbH) by analyzing 1000 binucleated cells in terms of the number of micronuclei (MN), dicentric bridges (DB) and nuclear buds (NB). The nuclear division index (NDI) was calculated according to the formula *NDI* = (1 × *MOC*) + (2 × *BC*) + (3 × *MUC*)/*N,* in which MOC is the number of mononucleate cells, BC - the number of binucleate cells, MUC - the number of multinucleated cells, and N - the total number of cells included. Table 11 shows the results of the tests performed, which indicate that the tested compounds do not show genotoxicity.

**Table 10. Results of cytotoxicity assessment in CHO-K1 cells in the MTT test performed for the tested compounds.**

| **Compound** | **Concentration [µM]** | **% Viability [mean]** | **SD** |
|---|---|---|---|
| **Control** | - | 100.0 | 8.3 |
| **A-111** | 200 | 98.6 | 5.9 |
| | 150 | 95.6 | 5.5 |
| | 100 | 86.1 | 7.5 |
| **A-133** | 200 | 89.1 | 16.6 |
| | 150 | 97.2 | 1.0 |
| | 100 | 94.6 | 1.3 |
| **A-67** | 200 | 84.0 | 4.5 |
| | 150 | 90.3 | 3.4 |
| | 100 | 96.0 | 8.9 |
| **H-425/21** | 200 | 88.0 | 1.6 |
| | 150 | 83.8 | 3.9 |
| | 100 | 87.6 | 5.1 |
| **A-102** | 200 | 87.2 | 3.3 |
| | 150 | 69.9 | 2.2 |
| | 100 | 84.7 | 6.7 |
| **H-415/21** | 200 | 87.2 | 3.9 |
| | 150 | 102.8 | 0.5 |
| | 100 | 102.2 | 1.1 |

**Table 11. Results of genotoxicity assessment of the tested compounds in CHO-K1 cells (number of micronuclei (MN), dicentric bridges (DB) and nuclear buds (NB) in 1000 binucleate cells and nuclear division index (NDI) .**

| **Tested sample** | **Concentration [µM]** | **MN** | **DB** | **NB** | **NDI** |
|---|---|---|---|---|---|
| **Negative control** | - | 8.3 ± 3.0 | 4.0 ± 2.2 | 4.8 ± 2.9 | 1.56 |
| **Positive control** | - | 93.0 ± 7.4 * | 23.5 ± 5.3 * | 26.0 ± 5.1 * | 1.27 * |
| | 100 | 4.5 ± 3.5 | 2.5 ± 0.7 | 2.0 ± 2.8 | 1.60 |
| **A-111** | 150 | 9.0 ± 5.7 | 4.0 ± 2.8 | 6.0 ± 1.0 | 1.64 |
| | 200 | 10.0 ± 4.2 | 2.0 ± 2.8 | 2.5 ± 0.7 | 1.64 |
| | 100 | 7.8 ± 2.9 | 3.8 ± 2.8 | 6.3 ± 3.7 | 1.64 |
| **A-133** | 150 | 6.0 ± 1.9 | 3.7 ± 2.3 | 4.5 ± 3.3 | 1.65 |
| | 200 | 6.8 ± 3.2 | 3.3 ± 1.4 | 4.7 ± 1.8 | 1.66 |
| | 100 | 12.0 ± 5.7 | 3.3 ± 0.6 | 5.0 ± 2.8 | 1.58 |
| **A-67** | 150 | 8.0 ± 2.8 | 2.3 ± 0.6 | 5.5 ± 2.1 | 1.62 |
| | 200 | 6.5 ± 2.1 | 3.0 ± 2.8 | 5.5 ± 0.7 | 1.62 |
| | 100 | 11.0 ± 4.2 | 2.5 ± 0.7 | 3.5 ± 0.7 | 1.56 |
| **H-425/21** | 150 | 11.5 ± 0.7 | 2.5 ± 2.1 | 3.5 ± 0.7 | 1.52 |
| | 200 | 12.5 ± 6.4 | 4.5 ± 3.5 | 5.5 ± 0.7 | 1.60 |
| | 100 | 10.5 ± 2.1 | 4.0 ± 2.8 | 5.5 ± 2.1 | 1.54 |
| **A-102** | 150 | 6.5 ± 2.1 | 2.5 ± 2.1 | 6.5 ± 2.1 | 1.54 |
| | 200 | 8.5 ± 0.7 | 3.0 ± 1.4 | 4.0 ± 1.4 | 1.60 |
| | 100 | 12.5 ± 0.7 | 1.5 ± 0.7 | 5.0 ± 1.4 | 1.54 |
| **H-415/21** | 150 | 12.0 ± 2.8 | 3.5 ± 2.1 | 5.5 ± 2.1 | 1.50 |
| | 200 | 11.5 ± 3.5 | 2.5 ± 0.7 | 6.0 ± 1.4 | 1.52 |

| | | | | | |
|---|---|---|---|---|---|
| Negative control - DMSO 0.1%, positive control - mitomycin C 0.5 µg/mL. Results are reported as mean ± standard deviation, (*) *p* < 0.05 compared to the negative control | | | | | |

### d) phototoxicity assessment using the Balb/c 3T3 murine fibroblast cell line

Balb/c 3T3 cells, which are an immortalized murine fibroblast cell line, were purchased at ATTC and cultured in the medium recommended by the manufacturer (DMEM GlutaMAX, newborn calf serum (BCS), antibiotics). After multiplication, cells were placed in 96-well plates, which were incubated for 24 hours, after which all wells of the plates were washed with 150 µL of balanced salt solution (HBSS). Then, after drying the plates, 100 µL of a solution of the tested compounds in HBSS with 2% FBS was added to each well and incubated for an hour. After this time, some of the plates were irradiated using the Suntest CPS+ sunlight simulator from Atlas with appropriate filters cutting off radiation with a wavelength <320 nm (UVA radiation intensity 5 J/cm²). The second part of the plates was placed in radiation-free conditions for the same period of time. After the exposure time, the liquid was removed from all plates and the wells were washed twice with 150 µL of HBSS, then supplemented with fresh culture medium and incubated for 24 hours in an incubator (37 °C, 5% CO₂). Viability was assessed in the neutral red uptake test, which is based on the ability of the dye to penetrate only into living cells. Dye uptake was confirmed by absorbance measurement at 540 nm. The obtained results indicate that compounds **A-111** and **A-133,** both as a result of exposure to UVA and in the absence of exposure, are devoid of cytotoxicity towards the mouse fibroblasts used in the study. The appropriate coefficients were also calculated: mean phototoxic effect (MPE) and photoirritation factor (PIF). PIF is the ratio of the IC₅₀ values of unirradiated to irradiated cells, while MPE is based on the comparison of concentration-response curves. The obtained values are presented in Table 12. The test results clearly showed that the tested compounds **A-111** and **A-133** do not have phototoxic potential. The mean phototoxic effect (MPE) values obtained in the experiment were less than 0.1, indicating no phototoxic effect. However, the results obtained for chlorpromazine, which is a positive control in the study, clearly indicate its phototoxic potential.

**Table 12. Phototoxicity of compounds A-111, A-133 and chlorpromazine.**

| **Tested compound** | **IC₅₀ [µg/mL]** | **MPE** | **PIF** |
|---|---|---|---|
| **A-111** | Not determined | 0.080 | No phototoxicity |
| **A-133** | Not determined | -0.013 | No phototoxicity |
| **Chlorpromazine** | - UV 18.56 | 0.499 | 25.618 |
| | + UV 0.726 | | |

### II. Tests of formulations containing exemplary compounds according to the invention

### 1. Composition and preparation of an example formulation.

A cosmetic formulation was prepared with the composition given below, containing the tested compound **A-111** at a concentration of 0.74%.

| **Ingredient:** | **Weight [g]:** |
|---|---|
| Water | 18.29 |
| PEG8 | 18.29 |
| Cetyl Alcohol | 3.0 |
| Isopropyl Palmitate | 3.0 |
| Simmondsia Chinensis Seed Oil | 2.0 |
| Butyrospermum Parkii Butter | 1.95 |
| Ceteareth-20 | 1.425 |
| Glyceryl Stearate | 1.075 |
| Phenoxyethanol | 0.45 |
| A-111 | 0.37 |
| BHT | 0.05 |
| Ethylhexylglicerin | 0.05 |
| Xanthan Gum | 0.05 |

The formulation was prepared in the following steps:
1. The ingredients of the oil phase (Isopropyl Palmitate, Simmondsia Chinensis Seed Oil, Butyrospermum Parkii Butter, Cetyl Alcohol), emulsifiers (Ceteareth-20, Glyceryl Stearate) and BHT were weighed - heated to 70 °C and mixed.
2. A solution of A-111 in PEG8 (=PEG400) with water was prepared, heated to 70 °C, the preservative Phenoxyethanol and Ethylhexylglycerin were added and mixed.
3. The fat phase was added to the water phase while mixing with a blender.
4. Xanthan Gum was added.
5. Homogenized.

The compositions obtained in this way were subjected to further biological tests.

### Inhibition of melanogenesis in a model of pigmented reconstructed human epidermis.

The research was carried out on reconstructed human epidermis containing melanocytes MelanoDerm^{™} MEL-300-B from MatTek (Bratislava, Slovakia). The ratio of keratinocytes to melanocytes in the epidermis used is 10:1. Only media purchased from the same manufacturer (EPI-100-NMM-113) was used to culture the epidermis during the experiments. Once the epidermis was obtained, it was transferred to 12-well plates containing 5 mL of medium/well. Plates were used to enable proper contact of the lower parts of the inserts with the substrate (so-called hanging-top plate). Solutions of the compounds were prepared immediately before their application. The solutions were applied to the inserts using a positive displacement pipette and then distributed using a sterile glass bulb headed Pasteur pipette. The application was repeated every 48 hours, a total of 7 applications were performed. Before the next application, each insert was rinsed with sterile DPBS and dried with a sterile cotton bud. The application was also accompanied by a change of the medium. All work during epidermis culture was carried out in a laminar air flow chamber, using aseptic principles and sterile reagents and utensils. The epidermis was cultured in an incubator ensuring appropriate humidity, temperature (37 °C) and CO₂ concentration (5%).

The experiment was conducted for 14 days. After this time, the inserts were rinsed with DPBS and dried with a cotton bud. For the selected pool of inserts, tissue viability was assessed using the MTT (Thiazolyl Blue Tetrazolium bromide) test, the remaining ones were used to determine the amount of melanin. To assess viability, the inserts were transferred to 24-well plate containing 0.3 mL of DMEM medium with 1 mg/mL MTT and incubated for 3 hours in an incubator. The epidermis was then placed in 2 mL of isopropanol and the resulting formazan was extracted for 2 hours at room temperature. The absorbance of the samples was measured at 570 nm in a microplate reader and correlated with the absorbance of the control by the formula % Viability = [OD (570 nm) for test compound/OD (570 nm) for control] × 100. To determine the amount of melanin, the inserts were incubated in 5% sodium bicarbonate for 30 min, then dried and placed in 0.5 mL of Solvable^{™} reagent, which caused complete lysis of the epidermis. After heating at 95 °C, the absorbance of the samples was measured at 405 nm in a microplate reader. On this basis, the inhibition of the amount of melanin was calculated in relation to the control sample. The absorbance results were also correlated with a standard curve for synthetic melanin. Table 13 shows the viability and melanin content results calculated from the absorbance measurements.

**Table 13. Results of melanogenesis inhibition and safety studies in the EpiDerm model.**

| **Tested sample** | **Melanin content [% control mean±SD]** | **Viability [% control mean±SD]** |
|---|---|---|
| **PEG 50% (25 µL)** - **control** | 100.00±10.20 | 100.00±2.43 |
| **A-111 formulation (25 µL)** | 62.54±9.36 | 130.91±2.66 |

### Phototoxicity studies in a model of reconstructed human epidermis.

The phototoxicity test of the prepared formulation containing the **A-111** compound was performed in a model of reconstituted human epidermis EpiDerm from MatTek. An epidermis with an area of 0.6 cm² was used. After receiving the tissue, the inserts were transferred to fresh medium and incubated in an incubator (5% CO₂, 37 °C). All work during epidermis culture and application of the tested compounds and formulations was carried out in a laminar air flow chamber, using aseptic principles and sterile reagents and utensils. To conduct the experiment, two 6-well plates were prepared, on which inserts were placed and the test substances were applied: 50 µL of sterile water (2 repetitions), 50 µL of 0.01% chlorpromazine solution (2 repetitions), 25 µL of A-111 formulation. The applied solutions and formulation were spread on the surface of the inserts using a sterile glass bulb headed Pasteur pipette. The plates were incubated in the incubator for 21 hours, after which the inserts were transferred to new 6-well plates with 0.9 mL DBPS. 1 plate was exposed to UVA radiation at a dose of 6 J/cm² using a solar light simulator (Solar Simulator, Atlas) as its source (+UVA plate). The second plate was placed in a dark place outside the incubator for the same period of time (-UVA plate). After exposure, inserts from both plates were rinsed with DPBS, dried with a sterile cotton bud and placed in new plates containing 0.9 mL of fresh growth medium and incubated for 21 hours. After this time, the MTT test was performed by placing the inserts in 0.3 mL of MTT solution in DMEM medium (concentration 1 mg/mL) for 3 hours. After this time, the formed formazan was extracted into isopropanol and the absorbance of all samples was read at 270 nm. Based on the results presented in Table 14, it can be concluded that the tested formulation did not show phototoxicity to the reconstructed human epidermis. The standard substance used - chlorpromazine at a concentration of 0.01% was phototoxic, which confirms the correct choice of the conditions of the experiment.

**Table 14. Viability of epidermal cells in the formulation phototoxicity test.**

| **Tested sample** | **Viability [% relative to water] ± SD** | |
|---|---|---|
| | -UVA | + UVA |
| **Water** | 100.00±5.99 | 100.00±4.72 |
| **Chlorpromazine 0,01%** | 99.52±9.41 | 26.97±6.05 |
| **A-111 formulation** | 93.4±1.38 | 97.15±3.11 |

### Permeation studies in reconstructed human epidermis.

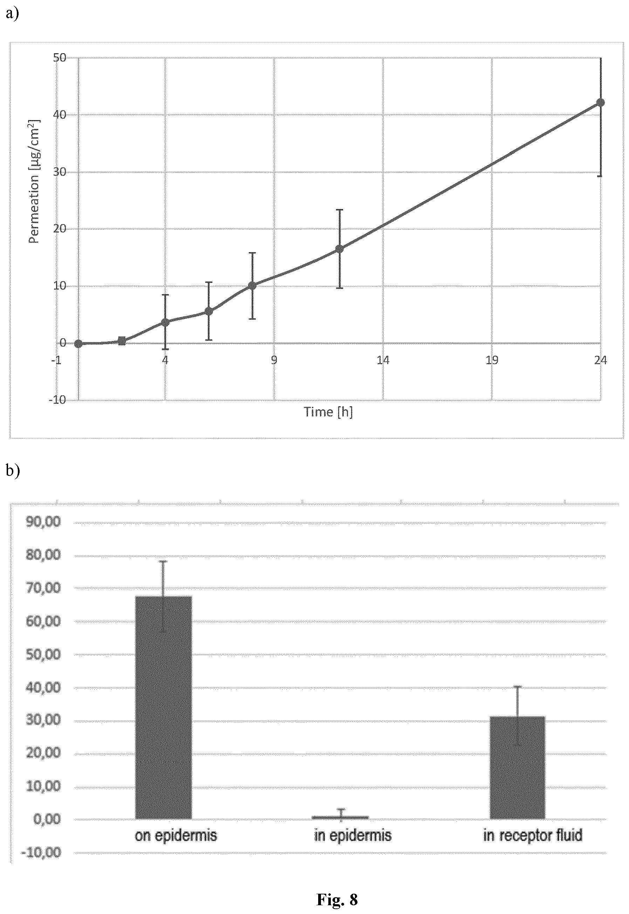

Permeation studies of the **A-111** compound from a cosmetic formulation were performed in a model of reconstructed human epidermis. Human reconstructed epidermis Episkin^{™} with an area of 1.07 cm² (Episkin, Lyon, France) was used for this purpose. Immediately after receipt, the tissue was transferred to fresh medium and cultured in an incubator (37 °C, 5% CO₂) for 18 hours. Before performing the experiments, the inserts were washed with sterile PBS and placed in the 12-well plate containing 2 mL of acceptor medium (PBS+0.25% Tween 20). 25 µL of the formulation containing the tested compound **A-111** was applied to the epidermis. The finished experimental plate was incubated at 32 °C. At specific time intervals, aliquots of the receptor fluid (0.5 mL) were collected for the analysis and replaced with 0.5 mL of fresh receptor fluid. After 24 h of culture, all of the receptor fluid was collected. The examined tissue was washed with cotton buds soaked in methanol. The donor and receptor compartments were washed with methanol, and the epidermis was separated from the inserts and transferred to methanol. The content (concentration) of the tested substance **A-111** in the receptor fluid and in individual compartments: on the surface of the epidermis, in the epidermis, in the receptor fluid was determined using measurements in a microplate reader at 281 nm on 96-well plates transmitting ultraviolet radiation. The study showed that the compound **A-111** released from the tested formulation crosses the epidermal barrier and has the potential to penetrate the dermis (Fig. 8). Fig. 8 shows the penetration of the compound **A-111** from the tested formulation (a) through the epidermis and the distribution [%] of compound **A-111** in the reconstructed human epidermis 24 hours after application of the tested formulation (UV analysis).

### Literature

[1] D.L. Stulberg, N. Clark, D. Tovey, Common hyperpigmentation disorders in adults: Part I. Diagnostic approach, café au lait macules, diffuse hyperpigmentation, sun exposure, and phototoxic reactions., Am. Fam. Physician. 68 (2003) 1955-1960.
[2] D.L. Stulberg, N. Clark, D. Tovey, Common hyperpigmentation disorders in adults: Part II. Melanoma, seborrheic keratoses, acanthosis nigricans, melasma, diabetic dermopathy, tinea versicolor, and postinflammatory hyperpigmentation., Am. Fam. Physician. 68 (2003) 1963-1968.
[3] D. Bandyopadhyay, Topical treatment of melasma. Indian J Dermatol. 54 (2009) 303-309.
[4] S.R. Desai, Hyperpigmentation therapy: a review. J Clin Aesthet Dermatol. 7 (2014) 13-17.
[5] S. Briganti, E. Camera, M. Picardo, Chemical and instrumental approaches to treat hyperpigmentation., Pigment Cell Res. 16 (2003) 101-110.
[6] Y. Shi, Q.X. Chen, Q. Wang, K.K. Song, L. Qiu, Inhibitory effects of cinnamic acid and its derivatives on the diphenolase activity of mushroom (Agaricus bisporus) tyrosinase, Food Chem. 92 (2005) 707-712.
[7] Y.H. Hu, X. Liu, Y.L. Jia, Y.J. Guo, Q. Wang, Q.X. Chen, Inhibitory kinetics of chlorocinnamic acids on mushroom tyrosinase, J. Biosci. Bioeng. 117 (2014) 142-146.
[8] Y.K. Seo, S.J. Kim, Y.C. Boo, J.H. Baek, S.H. Lee, J.S. Koh, Effects of p-coumaric acid on erythema and pigmentation of human skin exposed to ultraviolet radiation, Clin. Exp. Dermatol. 36 (2011) 260-266.
[9] J. Maignan, Cosmetic use of cinnamic acid derivatives as lightening agents. WO2004084854.
[10] S. Ullah, C. Park, M.Ikram, D. Kang, S. Lee, J.Yang, Y. Park, S. Yoon, P. Chun, H.R. Moon, Tyrosinase inhibition and anti-melanin generation effect of cinnamamide analogues, Bioorg. Chem. 87 (2019) 43-55.
[11] Q. Fan,, H. Jiang, E.D. Yuan, J.X. Zhang, Z.X. Ning, S.J. Qi, Q.Y. Wei, Tyrosinase inhibitory effects and antioxidative activities of novel cinnamoyl amides with amino acid ester moiety. Food Chem. 134 (2012) 134, 1081-1087.

## Claims

1. Compound of a formula 1: 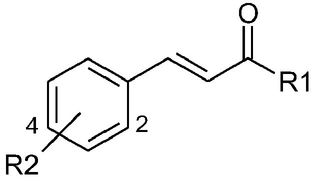
preferably with the E configuration of the double bond,
where:
R1 is an aminoalkanol, preferably: 6-aminohexan-1-ol, 5-aminopentan-1-ol, 4-aminobutan-1-ol, 3-aminopropan-1-ol, 2-aminoethan-1-ol, (*R*,*S*)-2-aminopropan-1-ol, (*R,S*)-2-aminobutan-1-ol, (*R,S)*-1-aminobutan-2-ol, (*R,S*)-2-amino-3-methylbutan-1-ol or 4-hydroxypiperidine,
R2 is a hydrogen atom, a chlorine atom, two chlorine atoms or a methyl group, at the appropriate position of the phenyl ring, preferably 4 or 2,4,
for use in the treatment or prevention of diseases or disorders related to abnormal melanogenesis, wherein the compound is used to inhibit melanogenesis or as a tyrosinase inhibitor.

2. The compound for use according to claim 1, **characterized in that** it is used in the treatment or prevention of skin discoloration, in particular for improving or unifying skin tone or in the treatment or prevention of hyperpigmentation, in particular chloasma, lentigines or postinflammatory, hormonal and solar hyperpigmentation.

3. The compound for use according to claim 1, **characterized in that** it is selected from the group comprising compounds of the formulas:

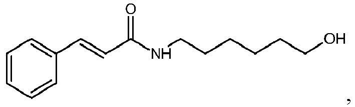

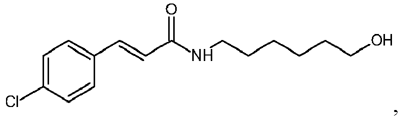

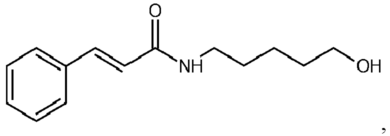

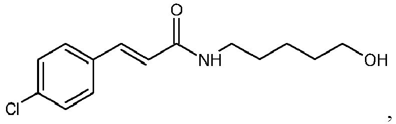

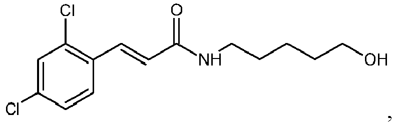

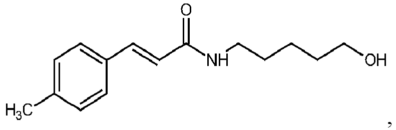

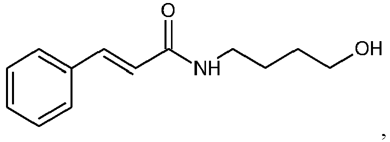

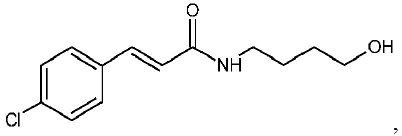

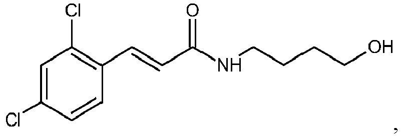

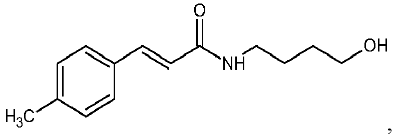

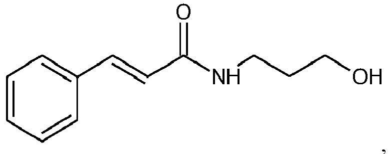

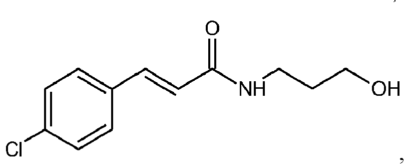

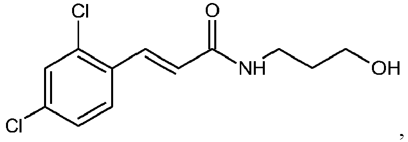

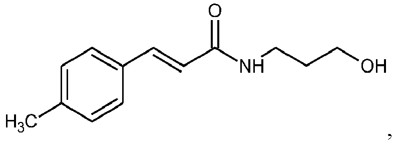

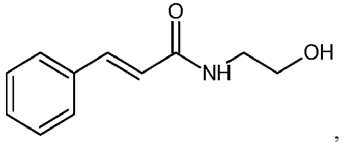

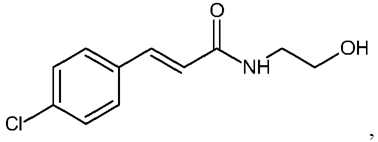

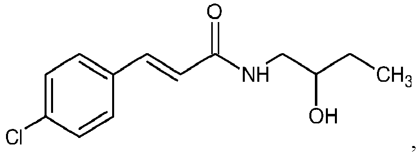

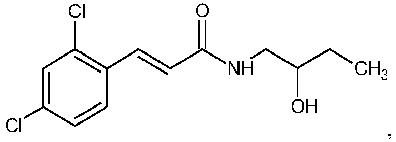

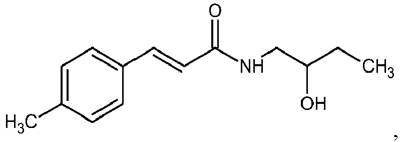

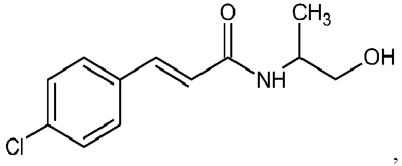

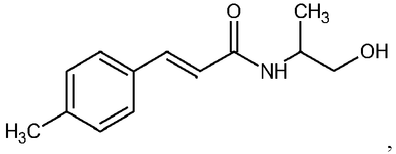

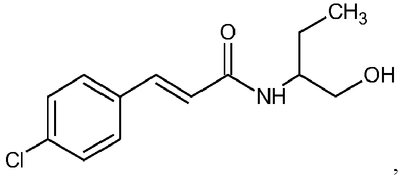

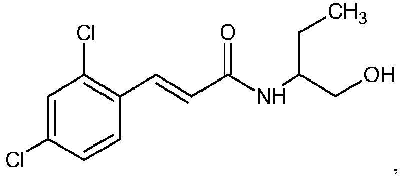

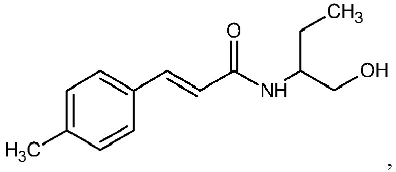

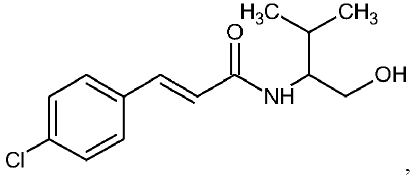

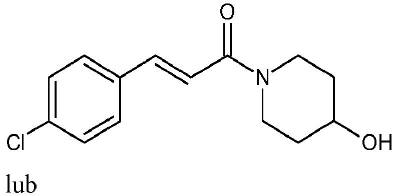

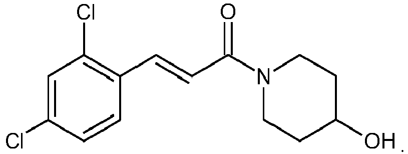

4. Use of the compound according to claim 1 or 3 as an ex-vivo tyrosinase inhibitor.

5. Use of the compound according to claim 1 or 3 in cosmetics.

6. Compound of formula 1 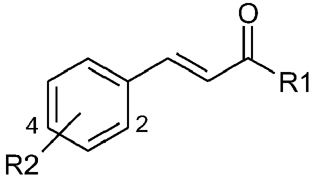
with *E* configuration of the double bond,
where:
R1 is an aminoalkanol and R2 is a hydrogen atom, a chlorine atom, two chlorine atoms or a methyl group,
selected from the group comprising:
(E)-*N*-(6-hydroxyhexyl)cinnamic acid amide (H-425/21),
(2*E*)-3-(4-chlorophenyl)-*N*-(6-hydroxyhexyl)prop-2-enamide (H-426/21),
(2*E*)-3-(2,4-dichlorophenyl)-*N*-(5-hydroxypentyl)prop-2-enamide (H-297/20),
(2*E*)-*N*-(5-hydroxypentyl)-3-(4-methylphenyl)prop-2-enamide (H-415/21),
(2*E*)-3-(4-chlorophenyl)-*N*-(4-hydroxybutyl)prop-2-enamide (H-331/21),
(2*E*)-3-(2,4-dichlorophenyl)-*N*-(4-hydroxybutyl)prop-2-enamide (H-298/20),
(2*E*)-N-(4-hydroxybutyl)-3-(4-methylphenyl)prop-2-enamide (H-411/21),
(2*E*)-3-(2,4-dichlorophenyl)-N-(3-hydroxypropyl)prop-2-enamide (H-296/20),
(2*E*)-*N*-(3 -hydroxypropyl)-3 -(4-methylphenyl)prop-2-enamide (H-410/21),
(2*E*)-3-(2,4-dichlorophenyl)-*N*-(2-hydroxybutyl)prop-2-enamide (H-320/21),
(2*E*)-3-(2,4-dichlorophenyl)-*N*-(1-hydroxybutan-2-yl)prop-2-enamide (H-285/20),
(2*E*)-3-(4-chlorophenyl)-*N*-(1-hydroxy-3-methylbutan-2-yl)prop-2-enamide (A-106),
(2*E*)-3-(2,4-dichlorophenyl)-1-(4-hydroxypiperidin-1-yl)prop-2-en-1-one (H-310/20),
(2*E*)-N-(1-hydroxybutan-2-yl)-3-(4-methylphenyl)prop-2-enamide (H-409/21) or
2*E*)-3 -(4-chlorophenyl)-1 -(4-hydroxypiperidin-1 -yl)prop-2-en-1 -one (A-99).
